(19) Europäisches Patentamt / European Patent Office / Office européen des brevets

(11) **EP 3 004 202 B1**

(12) **EUROPEAN PATENT SPECIFICATION**

(45) Date of publication and mention
of the grant of the patent:
**31.10.2018 Bulletin 2018/44**

(51) Int Cl.:
**C08G 63/664** (2006.01)   **A61K 9/51** (2006.01)
**A61K 31/765** (2006.01)

(21) Application number: **14736973.0**

(22) Date of filing: **28.05.2014**

(86) International application number:
**PCT/IB2014/061791**

(87) International publication number:
**WO 2014/191940 (04.12.2014 Gazette 2014/49)**

(54) **COPOLYMER AND NANOPARTICLES OBTAINED THEREFROM FOR DRUG DELIVERY**

COPOLYMER UND NANOPARTIKEL ZUR WIRKSTOFFFREISETZUNG DARAUS

COPOLYMÈRE ET NANOPARTICULES OBTENUES À PARTIR DE CELUI-CI ET DESTINÉES À ÊTRE UTILISÉES DANS L'ADMINISTRATION DE MÉDICAMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.05.2013 IT TO20130431**

(43) Date of publication of application:
**13.04.2016 Bulletin 2016/15**

(73) Proprietor: **Fondazione Istituto Italiano di Tecnologia**
**16163 Genova (IT)**

(72) Inventors:
• **GAGLIARDI, Mariacristina**
**I-16163 Genova (IT)**
• **BIFONE, Angelo**
**I-16163 Genova (IT)**
• **BERTERO, Alice**
**I-16163 Genova (IT)**

(74) Representative: **Casciano, Lidia Giulia Rita et al**
**Studio Torta S.p.A.**
**Via Viotti, 9**
**10121 Torino (IT)**

(56) References cited:
**WO-A1-03/093344    US-A- 5 132 397**

• **GAGLIARDI M; BARDI B; BIFONE A: "Polymeric nanocarriers for contolled and enhanced delivery of therapeutic agents to the CNS", THERAPEUTIC DELIVERY, vol. 3, no. 7, July 2012 (2012-07), pages 875-887, XP002719621, ISSN: 2041-5990 cited in the application**

Remarks:
The file contains technical information submitted after the application was filed and not included in this specification

EP 3 004 202 B1

**Description**

TECHNICAL FIELD

The present invention concerns a block copolymer and nanoparticles obtained therefrom. In particular, the nanoparticles are used as systems for drug delivery.

BACKGROUND ART

The systemic administration of active ingredients entails numerous problems which generally result in low effectiveness of the conventional pharmacological treatments. After the systemic administration of a drug, the response of the immune system generally causes a rapid and massive drop in the dose of drug circulating in the blood. It is therefore often necessary to administer higher doses than those sufficient to obtain the pharmacological effect.

Furthermore, numerous biological barriers are present in the organism specific for each organ which represent additional obstacles and limits to administration in specific tissues.

The capacity of a drug to cross the cell membrane and, subsequently, its distribution inside the cell are also fundamental for determining the pharmacological activity of the drug. For all these reasons, the possibility of using specific multifunctional carriers to protect the active molecules, cross the biological barriers and promote the internalization thereof has been carefully studied over the years. In the development of carriers with nanometric dimensions (nanocarriers), the possibility of using polymer materials has aroused great interest. Polymers are extremely versatile in terms of chemical-physical and functional properties and are highly adaptable to technical needs.

The use of polymeric nanocarriers offers many benefits. The drugs delivered by means of polymeric nanocarriers generally show a greater bioavailability than the therapeutic agents commonly administered on their own[1] with an increase in the therapeutic index and a reduction in side effects[2]. Furthermore, they can be used for the preparation of multifunctional systems[3,4] for transport and release of the drug at the target site[5,6] or in therapies based on the administration of several drugs simultaneously[7-9].

Further advantages of the use of polymeric nanocarriers for the administration of drugs are correlated with their ability to increase the solubility of poorly soluble drugs[10,11] and the stability of some therapeutic agents, such as peptides and oligonucleotides. Intelligent functional nanocarriers can be successfully used in the release of drugs to organs and tissues that cannot be easily reached by conventional therapies, like the central nervous system (SNC), protected by the hematoencephalic barrier (HEB)[12,13]. The properties required of a polymer for it to be usable as a nanocarrier for systemic administration are numerous. Of these, dimension is one of the most important. In particular, carriers with a diameter of around 1000 nm cannot be used because they do not have the capacity to be transported by intercapillarity and are not efficiently internalized in the endothelial cells that cover the blood vessels.

In addition, an efficient nanocarrier must be able to avoid or limit absorption by the mononuclear phagocyte system (MPS) (mimetism). In fact, after intravenous injection, the nanoparticles can be rapidly eliminated from the blood in a few minutes by the MPS and, in particular, by the Kupffer cells[14] with consequent failure of the treatment.

To increase the circulation time, nanoparticles with small dimensions can be used (with diameter of less than 100 nm)[15]. The mimetism can also be obtained by means of passivation of the surface using, for example, a hydrophilic shell or a distribution of hydrophilic polymeric blocks, such as poly(ethylene glycol) (PEG)[16,17], exposed to the external environment. PEG is a chemically inert linear polyether with low immunogenicity[18].

Numerous studies are reported in literature which analyse the effect of conjugation with PEG (PEGylation) on the absorption of blood proteins, on the uptake of the macrophages and on the circulation time[19-21] with interesting results. The nanoparticles conjugated with PEG are generally considered resistant to the macrophages due to the reduced, or even absent, surface opsonization, which leads to prolongation of the circulation times in the blood flow. This property is probably due to the increase in volume of the nanoparticle correlated with the surface absorption of the water which reduces the sites exposed for phagocytosis of the proteins. However, the prolonged circulation of PEGylated nanoparticles in the blood cannot be attributed exclusively to a reduction in their absorption by the macrophages.

The mimetic behaviour of the PEGylated nanoparticles can also be explained by a steric prevention effect due to the presence of conjugated PEG molecules on the surface of said nanoparticles[22].

A promising approach that has been followed in the use of polymeric nanoparticles is linked to the possibility of preparing biodegradable nanocarriers to avoid accumulation in the target organs. The biodegradable materials can be degraded in the physiological environment to form small molecules that can be easily eliminated from cells and tissues. The aliphatic polyesters constitute a class of biodegradable and bioresorbable polymers with interesting characteristics for biomedical use. In particular, of these, poly(lactic acid) (PLA), poly(lactic-co-glycolic acid) (PLGA) and poly (caprolactone) (PCL) have already been approved by the FDA for commercial use. PCL was one of the first polymers used in the biomedical field due to its characteristic of biodegradability, bioresorbability, easy synthesis with the possibility of controlling its properties and its excellent compatibility if mixed with other different polymers, allowing modulation of the final properties. On the other hand, studies on the degradation rate of this polymer have reported very slow cell degradation and bioresorption processes. For these reasons, in numerous applications the PCL has been substituted by other polymers, for example PLGA, with faster degradation kinetics and consequently faster drug release kinetics.

Generally, the release of degradation products from polyester matrixes leads to the formation of an acid environment around the polymer. The drawback is that this can lead to phenomena of instability of the molecules transported by the polymer if they are sensitive to the pH, to serious inflammatory responses and, in some cases, to cyotoxicity if the degradation is faster than the clearance capacity of the tissues in removing these products.

From this point of view, materials with a slower degradation, like PCL, can be more useful in limiting these effects.

In recent years, therefore, numerous polymers have been developed for delivery of drugs with biodegradability characteristics in an attempt to solve the problems described.

For example, degradable block polymer systems of type A-B are known in which A and B have a different hydrophobic character, like those studied by Shin[41] and Baimark[42].

Shin describes a biodegradable copolymer in which the most hydrophilic block consists of methoxy poly(ethylene glycol) and the least hydrophilic block consists of poly($\varepsilon$-caprolactone).

The drawback is that the biodegradation kinetics of poly($\varepsilon$-caprolactone) based materials can be slow (up to 3-4 years) and studies on the intracellular reabsorption profiles show that only oligomers with molecular weight below 3 kDa can be completely reabsorbed at intracellular level.

Baimark, on the other hand, describes a biodegradable copolymer in which the most hydrophilic block consists of methoxy poly(ethylene glycol) and the least hydrophilic block consists of a caprolactone-lactic acid random copolymer. The drawback is that the presence of units of lactide produces, following degradation, acid molecules favouring the formation of inflammations which can be severe.

Of the polymers considered for the preparation of nanocarriers for drug release, studies are found in literature on poly($\square$-valerolactone) PEGylate, for the release of drugs and growth factors. The studies show that nanocarriers consisting of these materials are suitable for long-term release of the molecules contained and slow degradation kinetics.

Carriers consisting of poly(caprolactone), both PEGylated and non-PEGylated, to be used for drug release following systemic injection, were also taken into consideration. Due to their high hydrophobicity, these vectors have shown a marked tendency to absorption on their surface of various proteins present in the blood, with consequent reduction in circulation time following the injection.

WO 03/093344 A1 discloses blockcopolymers comprising two blocks A and B, wherein block A consists of poly(ethylene glycol) monomethylether and block B is a copolyester.

Due to the high level of hydrophilicity of the polymeric matrix, the slow drug degradation and release kinetics, both these materials seem to be poorly adapted to use in vivo since they could cause an accumulation of synthetic material at the site of the pathology and excessively slow release of the drug.

The need is therefore felt in the art for polymeric carriers with rapid degradation kinetics which do not cause inflammatory reactions in the patient.

DISCLOSURE OF INVENTION

The object of the present invention is therefore to provide a new biodegradable copolymer which is free from the drawbacks of the systems described above and which can be used as a pharmaceutical carrier.

Said object is achieved by the present invention, which concerns a block copolymer according to claim 1, the preparation method thereof according to claim 7 and the use thereof according to claim 6.

The present invention also relates to nanoparticles comprising the block copolymer according to claim 9, and compositions comprising the same according to claim 14.

BRIEF DESCRIPTION OF THE DRAWINGS

The present invention will now be described in detail with reference to the Figures of the accompanying drawings, in which:

- Figure 1 illustrates the FT-IR spectra relative to the starting copolymer (a) and to the carboxyfluorescein conjugated copolymer (b);
- Figure 2 illustrates the $^{1}$H-NMR spectra both of the carboxyfluorescein conjugated copolymer (b) and the native copolymer (a);
- Figure 3 illustrates the $^{13}$C-NMR spectra relative to the starting copolymer (a) and the carboxyfluorescein conjugated copolymer (b);
- Figure 4 illustrates the results of the GPC analyses for the native copolymer (a) and the carboxyfluorescein conjugated copolymer (b);

- Figure 5 illustrates the results of the SLS analyses for the native copolymer (a) and the carboxyfluorescein conjugated copolymer (b);
- Figure 6 illustrates the morphological analysis (SEM) of the nanoparticles of native copolymer (a), of carboxyfluorescein conjugated copolymer (b) and nanoparticles loaded with RhB (c);
- Figure 7 illustrates the results of the DLS analyses for the native copolymer nanoparticles;
- Figure 8 illustrates the pH variation as a function of the native copolymer nanoparticle degradation time;
- Figure 9 illustrates the percentage weight loss as a function of the time observed during the degradation tests on native copolymer nanoparticles;
- Figure 10 illustrates the degradation constants of native copolymer nanoparticles obtained from the experimental data;
- Figure 11 illustrates the results obtained from the test for in vitro release of RhB in native copolymer nanoparticles (NPs) ;
- Figure 12 illustrates the results obtained with the tests for adsorption of BSA by native copolymer nanoparticles (NPs) ;
- Figure 13 illustrates the release of LDH from the bEND.3 cells after a treatment of 6h (a) and 24h (b) with the native copolymer nanoparticles. The panel c) illustrates the results of the cell metabolism measured with the WST-8 assay after 24 h;
- Figure 14 illustrates the results of the analysis with confocal microscopy of carboxyfluorescein conjugated copolymer nanoparticles after internalization by the bEND.3 cells after incubation for 6h (a), 24h (b), 48h (c) and 72h (d);
- Figure 15 illustrates the results of the analysis with confocal miocroscopy on bEND.3 cells treated with (a) nanoparticles loaded with RhB (10 $\mu$g/ml and (b) free RhB (113 ng/ml) after 6 h;
- Figure 16 illustrates the results of the analysis with confocal microscopy of the in vitro release of RhB in bEND.3 cells at a) 24 h, b) 48 h and c) 72 h after treatment with nanoparticles loaded with RhB (10 $\mu$g/ml) and colocalization with lysosomes;
- Figure 17 illustrates the % variation of the mean numeral molecular weight at different degradation times for the mPEG-b-(PCL-r-PVL) copolymer, in comparison with three commercial polymers;
- Figure 18 illustrates the pH variation of the degradation means obtained at various times.

## BEST MODE FOR CARRYING OUT THE INVENTION

According to a first aspect of the invention, a block copolymer is provided consisting of two blocks S1 and S2, in which S1 consists of methoxy poly(ethylene glycol) m(PEG)n, where n is an integer in the range between 4 and 50, and S2 is a random copolymer consisting of the monomer units R1 and R2 where R1 is the monomer unit corresponding to the monomer $\gamma$-valerolactone and R2 is the monomer unit corresponding to the monomer selected from the group consisting of $\epsilon$-caprolactone, $\delta$-valerolactone, $\beta$-butyrolactone, $\epsilon$-caprolactam and $\delta$-valerolactam.

In this text by "monomer unit" we mean the form subjected to reaction of a monomer into a polymer.

In one embodiment, R2 is the monomer unit corresponding to the $\epsilon$-caprolactone monomer.

The copolymer therefore consists of two blocks: the one consisting of m(PEG)n is hydrophilic whereas the one consisting of random copolymer consisting of R1 and R2 is hydrophobic. The chains of said copolymer in aqueous solution are arranged in a micelle type configuration in which the hydrophilic block is exposed towards the solution while the hydrophobic block is inside the micelle itself. Advantageously, the use of the $\gamma$-valerolactone monomer allows a reduction to be obtained in the molar enthalpy of fusion with respect to PCL and the poly $\delta$-valerolactone (PVL). This entails a lower crystallinity of the polymer obtained and therefore faster degradation kinetics in aqueous environment than many other aliphatic polyesters (the lesser packing at molecular level of the polymer favours the absorption of liquids and therefore biodegradability); promotes bioresorption (the degradation products are oligomers with low molecular weight, which can be easily metabolised and expelled from the organism, and not non-biodegradable crystalline fragments which could accumulate); and reduced mechanical properties (the polymer is less resistant to external mechanical stress, and this is an advantage in the case of biomedical devices consisting of biodegradable materials because the poor mechanical characteristics limit resistance to the osmotic phenomena which occur inside them, favouring the absorption of water and, consequently, the degradation kinetics). Furthermore an increase is observed in the vitreous transition temperature (Tg) as the fraction of $\gamma$-valerolactone contained in the copolymer of the invention increases. Also this characteristic influences the biodegradation: a Tg much lower than the external T can favour annealing of the device and, consequently, amorphous domains could be transformed into crystallines over time, causing the formation of fragments which cannot be eliminated in any way by the organism.

Lastly the degradation mechanism of the copolymer of the invention occurs by surface degradation. Therefore the profile of the pH in the surrounding medium is stabler over time than materials which undergo bulk degradation (polycaprolactone, polyvalerolactone, poly-3-hydroxybutyrate).

The molar ratio between R1 and R2 in the S2 block is between 5/95 and 30/70, in particular it is 20/80. Said range allows the thermal, mechanical and biodegradability properties of the copolymer to be widely modulated. For ratios greater than R1/R2 (up to 40/60) low reaction yields and molecular weights are obtained (yield < 30%, Mw < 5000 Da). For even higher ratios, only oligomers of ε-caprolactone (Mw < 3000 Da) can be obtained.

The block copolymer is characterised by having a molar ratio between the two blocks S1 and S2 of between 0.005 and 0.25, preferably between 0.038 and 0.064.

According to a preferred embodiment, the number of units of PEG varies between 12 and 45. In particular, the molecular weight of the methoxy poly(ethylene glycol) can be 550, 750 or 2000 Da.

The copolymer of the invention can be advantageously used in therapy or prophylaxis, for example as a pharmaceutical carrier.

According to a third aspect of the invention, a method of preparation of the block copolymer is also provided. Said method consists in reacting R1 and R2 in the presence of S1 and a catalyst selected from the list consisting of tin(II)-2-ethylhexanoate (Sn(Oct)$_2$), Al isopropoxide, Zn(II) lactate. In a preferred form, the catalyst used is tin(II)-2-ethylhexanoate, Sn(Oct)$_2$.

Advantageously said catalyst has already been approved for the synthesis of biomaterials by some government control bodies such as the FDA.

According to a fourth aspect of the invention, a nanoparticle is provided comprising the block copolymer. The particles thus obtained can be used to deliver a drug. The nanoparticle has a dimension in the range between 50 nm and 320 nm. Advantageously this dimensional range allows the nanoparticles of the invention to be transported by intercapillarity and to be efficiently internalized in the endothelial cells which cover the blood vessels. The dimensions of the nanoparticles subject of the invention can furthermore be less than 100 nm; this represents a further advantage since said nanoparticles have a longer circulation time than nanoparticles with larger dimensions.

In one embodiment, said nanoparticle furthermore comprises a drug. Said nanoparticles containing drugs are furthermore particularly advantageous since the m(PEG)n block gives the macrophages resistance, suppressing or reducing the opsonization of the nanoparticle by the proteins contained in the plasma, further increasing the circulation in the blood flow. The drug used for the production of said nanoparticles is selected from the group consisting of taxol, tacrolimus, everolimus, rapamycin, methotrexate and anthracyclines.

The drug is preferably present in quantities up to 30% by weight with respect to the copolymer.

According to a further aspect of the invention, a pharmaceutical composition is provided comprising the nanoparticles comprising the block copolymer and the drug.

Further characteristics of the present invention will become clear from the following description of some merely illustrative and non-limiting examples.

The following abbreviations are used in the examples reported below: min (minutes), h (hours), g (grams), mg (milligrams), μg (micrograms), ng (nanograms), mm (millimetres), μm (micrometres), nm (nanometres), mol (moles), mmol (millimoles), nmol (nanomoles) T$_m$ (melting temperature), T$_c$ (crystallisation temperature), eq (equivalents), 1 (litres), ml (millilitres), μl (microlitres), kDa (kilodalton), Da (dalton), mM (millimolar), nM (nanomolar), w/v (weight/volume), w/w (weight/weight), r.t. (room temperature), T$_g$ (vitreous transition temperature), rpm (rotations per minute), Mw (mean ponderal molecular weight), Mn (mean numeral molecular weight), CL (ε-caprolactone), VL (γ-valerolactone), mPEG (methoxy poly(ethylene glycol)), Sn(Oct)$_2$, (tin octoate or tin(II)-2-ethylhexanoate), N$_2$ (nitrogen gas), CH$_2$Cl$_2$ (or DCM, dichloromethane), CF (5(6)-carboxyfluorescein), CDCl$_3$ (deuterated chloroform), CH$_3$COCH$_3$ (dimethyl ketone), DMAP (4-(dimethylamino)pyridine), MeOH (methanol), THF (tetrahydrofuran), EtOH (ethanol), DCCI (N-(3-dimethylaminopropyl)-N'-ethylcarbodiimide chloride), RhB (rhodamine B), PBS (phosphate buffered saline), BSA (bovine serum albumin), FBS (fetal bovine serum), WST-8 (2-(2-methoxy-4-nitrophenyl)-3-(4-nitrophenyl)-5-(2,4-disulfophenyl)-2H-tetrazole), HPLC (high performance liquid chromatography), NMR (nuclear magnetic resonance), FTIR ATR (attenuated total reflectance Fourier transform infrared spectroscopy), GPC (gel permeation chromatography), SLS (static light scattering), DLS (dynamic light scattering), DSC (differential scanning calorimetry), SEM (scanning electron microscope), ACS (American Chemical Society), ROP (ring opening polymerisation), excitation wavelength (EX), emission wavelength (EM), standard error (SE).

Example 1

Synthesis of copolymer methoxy poly(ethylene glycol)-b-poly(ε-caprolactone-rand-γ-valerolactone) (mPEG-b-(PCL-r-PVL))

The ε-caprolactone (CL, Aldrich, Milan, Italy), the γ-valerolactone (VL, Aldrich) and the methoxy poly(ethylene glycol) (mPEG, Mn = 550 Da, Sigma Aldrich, Milan, Italy), were kept in a glass vial above molecular filters and were used as monomers and initiator respectively for synthesis via ring opening polymerisation (ROP). Tin(II)-2-ethylhexanoate (Sn(Oct)$_2$, tin octoate, Sigma) was used as the catalyst without any previous treatment. CL (31.42 mmol, 3.59 g), VL (7.85 mmol, 0.79 g) and mPEG (0.33 mmol, 0.18 g) were weighed so as to obtain a copolymer with a theoretical molecular weight of 14 kDa and a CL/VL molar ratio percentage of 80/20.

The reagents were placed in a flask which was then heated to 120°C using a thermostatic bath. Once the temperature had been reached, the catalyst was added (0.33 mmol, 0.13 g, molar ratio between initiator and catalyst 1:1); the reactor was fluxed with $N_2$ for 10 min and then sealed.

The reaction was maintained for 4 h at 120°C under bland stirring, then the reactor was rapidly cooled in a water bath to immediately stop the reaction, causing solidification of the product.

The product obtained, the copolymer methoxy poly(ethylene glycol)-b-poly($\varepsilon$-caprolactone-rand-$\gamma$-valerolactone) (mPEG-b-(PCL-r-PVL)) was dissolved in anhydrous $CH_2Cl_2$ (Sigma Aldrich) and precipitated in methanol (MeOH, Sigma, HPLC degree of purity) three times to eliminate the monomers that had not reacted and the catalyst. After the purification, the copolymer in powder is recovered by filtration and dried for 24 h in a ventilated stove: lastly it is conserved in a sealed glass vial at room temperature.

Example 2

Marking of the copolymer

The copolymer prepared as reported in example 1 was marked using 5(6)-carboxyfluorescein (CF, Sigma).

For this reaction, the copolymer (390.0 mg), 4-(dimethylamino)pyridine (DMAP, Aldrich, 0.01 mmol, 1.8 mg) and N-(3-dimethylaminopropyl)-N'-ethylcarbodiimide chloride (DCCl, Sigma Aldrich, 0.15 mmol, 28.7 mg) were dissolved in 10 ml of $CH_2Cl_2$.

When the mixture was uniform, CF was added (0.30 mmol, 113.0 mg) to the reaction and kept under stirring at room temperature (25°C) for 4 h. At the end of the reaction, the product (mPEG-b-(PCL-r-PVL)-CF) was purified by precipitation in MeOH three times and then dried and kept at -20°C. The conjugation reaction yield was evaluated by UV spectrometry. 10 mg of the marked copolymer were weighed and dissolved in acetone, 1 ml of the sample was then placed in a quartz cuvette and analysed.

The absorption data were recorded at 25°C with a JASCO V550 spectrometer (JASCO Europe, Cremello, Italy).

On the basis of the calibration curve of the CF solutions in acetone, from the absorbance values at 468 nm of the copolymer solution spectrum, the quantity of CF was identified and the ratio between CF and copolymer was evaluated.

The analysis was conducted on three copolymer solutions.

The degree of conjugation with the CF, expressed as %mol of CF/mol of copolymer, was 31.96 $\pm$ 6.16%.

Example 3

Physical-chemical characterisation of the copolymer according to the invention

The chemical characterisation of the native or marked copolymers was conducted by means of FTIR ATR, $^1$H-NMR and $^{13}$C-NMR analyses.

The FTIR-ATR analysis was conducted to define the chain structure of the material prepared and to verify whether the conjugation with CF had been successful; the FTIR spectra were recorded with a FTIR Cary 630 spectrometer (Agilent Technologies, Cernusco sul Naviglio, Italy) in the wavelength range of 4000-650 $cm^{-1}$.

The $^1$H-NMR and $^{13}$C-NMR spectroscopies were conducted with Varian Unity 400 spectrometer (300 Hz), using $CDCl_3$ as solvent (Aldrich, the sample concentration was 2% w/v).

The molecular weights of the products were evaluated by means of GPC, SLS and NMR.

For the GPC analyses, a Waters Alliance 2695 chromatograph equipped with a PDA (Waters 2995) and an RI detector, and a ResiPore column 300 x 7.5 mm (Agilent Technologies, Cernusco sul Naviglio, Italy) were used; THF (Aldrich, Chromasolv degree of purity) (1 ml/min) was used as the internal mobile phase, the system was kept at 30°C; the results were analysed on the basis of a calibration curve obtained from poly(styrene) monodisperse standards (Agilent Technologies, Cernusco sul Naviglio, Italy).

The SLS analyses were conducted using a Zetasizer ZS90 (Malvern Instruments, Malvern, UK); six solutions were analysed with concentration between 10 mg/ml and 3 mg/ml using toluene (Sigma Aldrich) as the standard and acetone as the solvent.

The analysis was conducted at 30°C and with a scattering angle of 90°; the tests were conducted in triplicate. For evaluation of the molecular weight by means of NMR spectra, the analysis was based on the terminal groups.

The resonance due to the protons of the methoxyl group was taken as a reference for evaluation of the mean numeral molecular weight and the molar composition of the material.

The thermal characterisation was conducted by means of DSC (DSC1 Mettler Toledo, Milan, Italy). 5 $\pm$ 0.003 mg of copolymer in powder were weighed and placed in sealed aluminium capsules. The analyses were conducted by applying a temperature ramp (10°C/min) from -65°C to 180°C under flux of gaseous $N_2$.

The thermograms of the second scan were normalised based on the weight of the sample and used to collect the data. The mid point of the heat flow slope change was taken as the vitreous transition temperature ($T_g$), and the specific heat difference ($\Delta c_p$) associated with $T_g$ was determined.

The melting temperatures ($T_m$) were taken as the minimum of the endothermic peaks while the crystallisation temperatures ($T_c$) were taken as the maximum of the exothermic peaks.

The wettability of the copolymer surface was evaluated by measurements of the static contact angle of the water on thin films obtained by means of spin coating. 100 $\mu$l of a polymeric solution in THF (10% w/v) were deposited on a flat glass support rotated at 600 rpm for 1 min, then at 1400 rpm for 2 min and lastly maintained on a plate heated to 40°C for 1 min to completely eliminate the residual solvent.

The contact angles were evaluated be depositing a drop of water (3 $\pm$ 0.5 $\mu$l) with a glass syringe on a flat polymeric surface, and the form of the drop and the contact angles were calculated by means of the Young-Laplace equation.

Three polymer samples were prepared and five measurements for each sample were taken.

Results

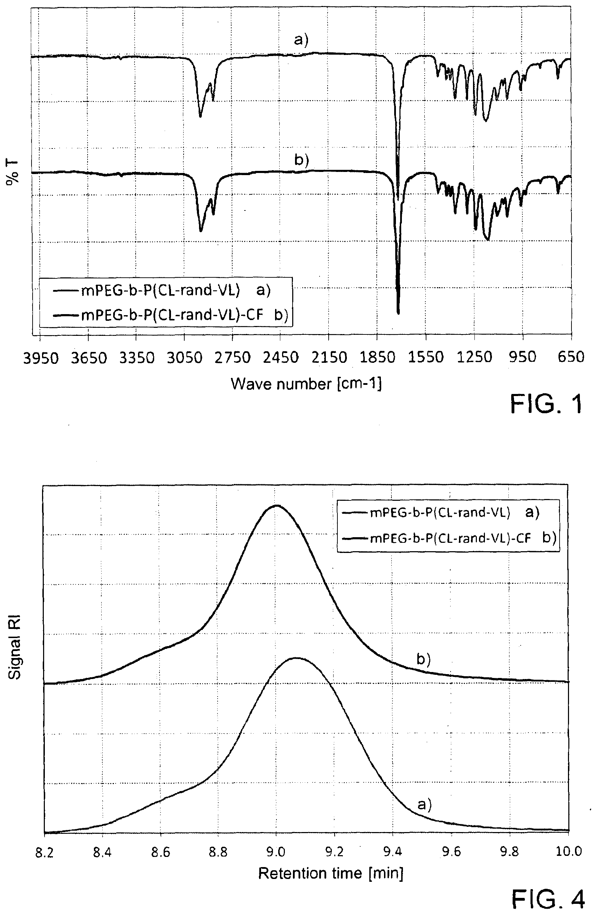

The chemical characterisation of the synthesised materials is summarised in Figures 1-3. Figure 1 illustrates the FT-IR spectrum relative to the starting copolymer (Figure 1a) which shows the following bands: 3600-3400 cm$^{-1}$ (stretching O-H), 3040-2730 cm$^{-1}$ (stretching -CH-), 1820-1600 cm$^{-1}$ (stretching of the C=O bond of the ester), 1430-1375 cm$^{-1}$ (-CH$_3$ due to bending of the VL), 1210-1118 cm$^{-1}$ (stretching of the CO-O bond of the ester), 1070-1015 cm$^{-1}$ (stretching of the C-O-C bond of the mPEG), 720-675 cm$^{-1}$ (bending of the aliphatic -CH$_2$-).

The CF conjugated copolymer, in addition to the characteristic bands of the native copolymer, shows a small shoulder at 1090 cm$^{-1}$ which can be attributed to the aromatic rings of the fluorescent colouring and the intensification of the stretching peak of the C-OH bond at 1161 cm$^{-1}$ (Figure 1b).

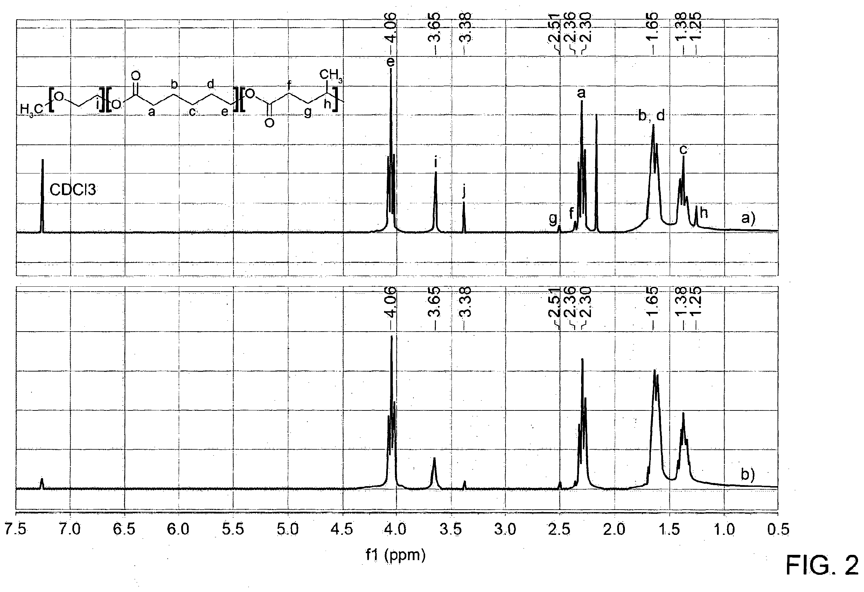

The $^1$H-NMR spectra both of the marked copolymer and the native copolymer show the characteristic resonances linked to the presence of the CL (see Figure 2, a: 2.30 ppm, b and d: 1.65 ppm, c: 1.38 ppm, and: 4.06 ppm), the VL (f: 2.36 ppm, g: 2.51 ppm and h: 1.25 ppm) and the mPEG (j: 3.38 ppm and i: 3.65 ppm). The resonance at 3.38 ppm was taken as the reference for evaluation of the molecular weight and molar composition of the copolymer. The effective molar composition of the copolymer random block was calculated considering the values of the integrals at 4.06 ppm (characteristic of the CL units), 1.25 ppm (typical of the VL units) and 3.65 ppm (attributed to the PEG chain). From this evaluation; the CL/VL ratio is 80.7/19.3.

The molecular weight $M_{n,NMR}$ was evaluated from the integrals. The results are reported in Table 1.

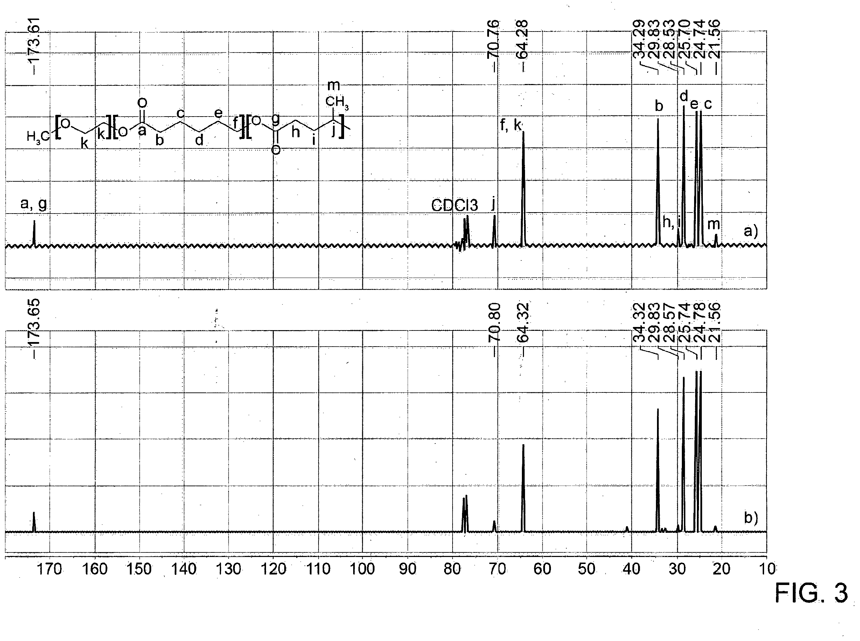

The $^{13}$C-NMR spectra show for both the materials resonances linked to the monomers used (see Figure 3, a and g: 173.61 ppm, f and k: 64.28 ppm, m: 21.56 ppm, c: 24.74 ppm, e: 25.70 ppm, d: 28.53 ppm, b: 34.29 ppm, j: 70.76 ppm, h and i: 29.83 ppm).

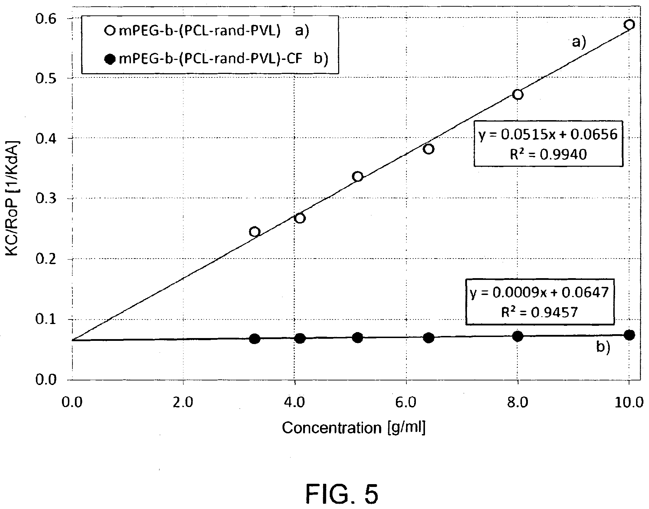

The molecular weights obtained from the GPC and SLS analyses both for the mPEG-b-(PCL-r-PVL) and for the mPEG-b-(PCL-r-PVL)-CF are reported in Figures 4 and 5 and the data obtained are summarised in Table 1.

Table 1.

|  | GPC analyses | | | NMR | SLS |
| --- | --- | --- | --- | --- | --- |
| Copolymer | Mn (kDa) | Mw (kDa) | PDI [Mw/Mn] | Mn (kDa) | Mw (kDa) |
| mPEG-b-(PCL-r-PVL) | 9.4 | 16.6 | 1.76 | 9.7 | 16.4 |
| mPEG-b-(PCL-r-PVL)-CF | 9.8 | 15.5 | 1.59 | 9.7 | 15.5 |

For evaluation of the molecular weight using the SLS technique, it is known that the relation between the intensity of the diffuse light and the molecular weight is given by the Rayleigh equation[23]:

$$\frac{K \cdot c}{R_\theta} = \frac{1}{M_w} + 2 \cdot A_2 \cdot c$$

where K is an optical constant depending on the differential increment of the refractive index, c is the concentration of the sample analysed, $R_\theta$ is the Rayleigh ratio, $M_w$ is the mean molecular weight and $A_2$ is the second virial coefficient.

From the data collected analysing the polymeric solutions with different concentrations, the Mw values were extrapolated for c->0 from a linear interpolation of the data while the value of $A_2$ was calculated from the intercept. The second virial coefficient represents an estimate of the interaction force between the molecules of the solute and the solvent. The values of $A_2$ found are $2.57 \cdot 10^{-2}$ ml·mol/g$^2$ and $4.71 \cdot 10^{-4}$ ml·mol/g$^2$ for the native copolymer and for the marked copolymer respectively.

The positive values of $A_2$ are associated with stable polymeric solutions without the tendency of the molecules to aggregate.

The data collected from the second scan of the DSC analysis show that the copolymer is semicrystalline with a melting temperature of 53.3±0.42°C and the specific melting enthalpy associated is 86.94±0.56 J/g; the marked copolymer shows a higher melting enthalpy (-81.96±2.73 J/g) at 53.15±0.61°C. Considering the crystallisation, the enthalpy associated with the native copolymer is 83.62±0.35 J/g and the associated temperature is 36.44±0.11°C; the marked copolymer shows a crystallisation phenomenon at 35.22±0.01°C with a crystallisation enthalpy of 80.61±0.26 J/g. Considering that the copolymer consists of a high percentage of caprolactone, the crystallinity percentage can be estimated with the following equation[24]:

$$x_c = \frac{\Delta H_m}{\Delta H_m^0} \cdot 100 \qquad (4)$$

in which $\Delta H_m$ represents the value of the melting enthalpy obtained from the DSC analyses and $\Delta H_m^0$ is the melting enthalpy of a crystalline sample consisting 100% of poly(caprolactone) corresponding to -139.3 J/g[24]. The results obtained for the non-marked copolymer and for the CF conjugated copolymer are 61.41 ± 0.40% and 58.83 ± 1.96% respectively.

The vitreous transition temperatures measured are -48.56 ± 2.88°C and -48.73 ± 2.79°C for the native copolymer and conjugated copolymer respectively with an $\Delta c_p$ of 2.61 ± 0.15 and 2.91 ± 0.19 J/g·K.

The static contact angle evaluated on a thin polymeric film of the native copolymer was 84.06 ± 0.38°. This value is very similar to those reported in literature for the homopolymers of poly (caprolactone)[26, 27].

Example 4

Synthesis of nanoparticles comprising the copolymer according to the invention

The nanoparticles were obtained with the technique of solvent shifting in the absence of surfactants.
10 mg of the copolymer mPEG-b-(PCL-r-PVL) were dissolved in 2 ml of dimethyl ketone ($CH_3COCH_3$) and added dropwise in a beaker containing 20 ml of ethanol (EtOH) at room temperature and under vigorous mechanical stirring (1300 rotations per minute (rpm)). After 1 h the temperature was increased to 40°C for 30 min so as to guarantee complete evaporation of the $CH_3COCH_3$ and obtain a suspension of mPEG-b-(PCL-r-PVL) nanoparticles in ethanol.

Example 5

Synthesis of nanoparticles comprising the copolymer and a drug according to the invention

30 mg of the copolymer mPEG-b-(PCL-r-PVL) and 3 mg of rhodamine-B (RhB) were dissolved in 6 ml of dimethyl ketone ($CH_3COCH_3$) and added dropwise in a beaker containing 30 ml of ethanol (EtOH) at room temperature and under vigorous mechanical stirring (1300 rpm). After 1 h the temperature was increased to 40°C and maintained for 30 minutes so as to guarantee complete evaporation of the dimethyl ketone. The nanoparticles were recovered from the reaction medium by means of filtration on paper and washing with distilled water so as to eliminate the RhB not trapped in the particles. The encapsulation efficiency was evaluated from calculation of the RhB still in solution: 10 μl of the reaction medium were withdrawn, diluted in distilled water and analysed by means of fluorescence spectroscopy with a Cary Eclipse fluorometer (Varian, Paolo Alto, CA). The encapsulation efficiency (%EE) was calculated with the following formula:

$$\%EE = \frac{q_{RhB.f}}{q_{RhB.0}} * 100 \qquad (1)$$

where $q_{RnB.0}$ is the initial quantity of RhB dissolved in the solution, and $q_{RhB.f}$ is the quantity of RhB incorporated in the particles, calculated as the difference between $q_{RhB.0}$ and the quantity of RhB present in the solution at the end of the encapsulation process.

The encapsulation efficiency percentage of RhB evaluated with the equation 1 was 11.4%, corresponding to 11.3 ng/μg of polymeric nanoparticles (1.1% w/w).

Example 6

Morphological and dimensional characterisation of the nanoparticles

The morphology of the nanoparticles of native copolymer and of copolymer conjugated and loaded with RhB was observed by means of scanning electron microscope SEM. The nanoparticles in ethanol were deposited on silica and the solvent was left to evaporate before placing the sample in the microscope.

Evaluation of the dimensions of the nanoparticles in a liquid medium and the Z potential was performed by using a Zetasizer instrument (ZS90, Malvern). 1 ml of water, previously filtered with a filter with cut off of 0.2 $\mu$, was added to 30 $\mu$l of the suspension of nanoparticles in ethanol.

Three samples were prepared for each preparation and five measurements were taken for each sample.

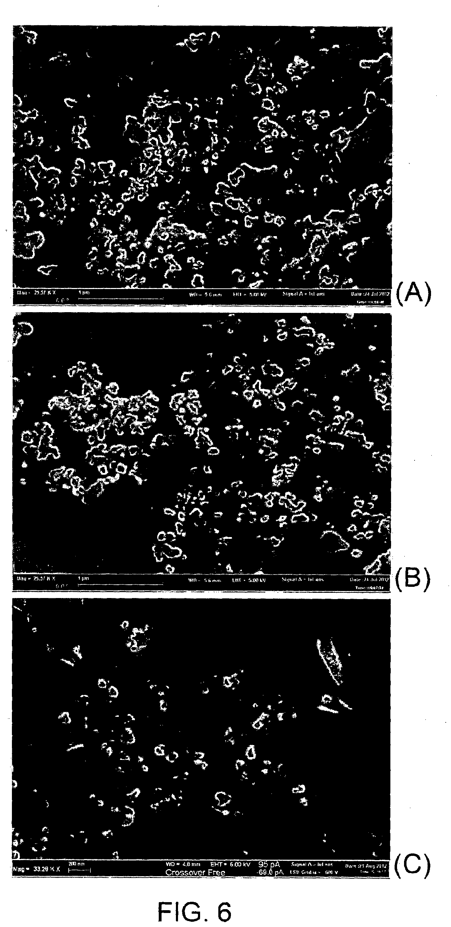

Figure 6 shows the results of the morphological analysis conducted with SEM. Using the ImageJ software, the SEM images were analysed to evaluate the mean diameter of the nanoparticles. The data obtained are summarised in Table 2 and compared with the results obtained with the DLS analysis.

Table 2

|  | mPEG-b-(PCL-r-PVL) (a) | mPEG-b-(PCL-r-PVL)-CF (b) | mPEG-b-(PCL-r-PVL)/RhB (c) |
|---|---|---|---|
| SEM (nm) | 65.8 $\pm$ 17.2 | 69.2 $\pm$ 21.8 | 75.2 $\pm$ 22.8 |
| DLS (nm) | 59.2 PDI = 0.17) | 61.7 (PDI = 0.28) | n.a. |

The potential z of the nanoparticle solutions was also evaluated. The results obtained are: -12.53 $\pm$ 0.12 mV and - 21.67 $\pm$ 0.37 mV for the native copolymer nanoparticles and for the CF conjugated nanoparticles respectively.

Example 7

Functional characterisation

To evaluate the stability and hydrophilicity of the nanoparticles, a swelling test was conducted which measured by means of DLS the increase in diameter of the nanoparticles after different incubation times in a phosphate buffer (PBS, pH 7.4) at 37°C.

30 $\mu$l of a solution of fresh nanoparticles were added to 1 ml of PBS solution in a plastic test tube and incubated at 37°C. At the established times, the cuvettes were analysed by DLS at 37°C. The tests were conducted in triplicate.

The degradation tests were conducted by immersing 100 $\mu$g of nanoparticles in 500 $\mu$l of degradation medium. The tests were performed in water, PBs solution (pH 7.4) and Dulbecco's Modified Eagle Medium (DMEM). Serum was furthermore added to the culture.

The degradation means was selected considering analysis of the degradation behaviour of the nanoparticles.

The samples were kept at 37°C in a thermostatic bath, separated at different times and centrifuged, the degradation medium was collected to evaluate the pH variation while the nanoparticles were dried at room temperature; lastly the degraded nanoparticles were dissolved in THF and analysed by GPC to observe the molecular weight reduction during the degradation tests. The tests were conducted in triplicate.

For the rhodamine release tests, 50 $\mu$g of loaded nanoparticles suspended in ethanol were added to 1 ml of PBS solution (pH 7.4) and kept at 37°C for the entire duration of the test (3 days). At set times (6, 24, 48 and 72 h), the samples were centrifuged and the release medium was separated and analysed by fluorescence spectroscopy to determine the quantity of rhodamine released by the nanoparticles. The test was conducted in triplicate.

The nanoparticles prepared using the native copolymer were used to analyse the quantity of bovine serum albumin (BSA, Sigma) adsorbed on the surface of the nanoparticles. 1 mg of nanoparticles suspended in EtOH were added to a solution of BSA in PBS (0.5 mg/ml) . At set times (0.5, 1, 2, 3.5, and 6 h) the samples were centrifuged and the medium was separated and analysed by spectrophotometry to evaluate the quantity of BSA adsorbed during the test. The analyses were conducted in triplicate.

Results

The swelling tests showed that the nanoparticles rapidly increase their diameter in the first part of the test (90 min) until they reach a plateau value which is maintained for the remaining time of the experiment.

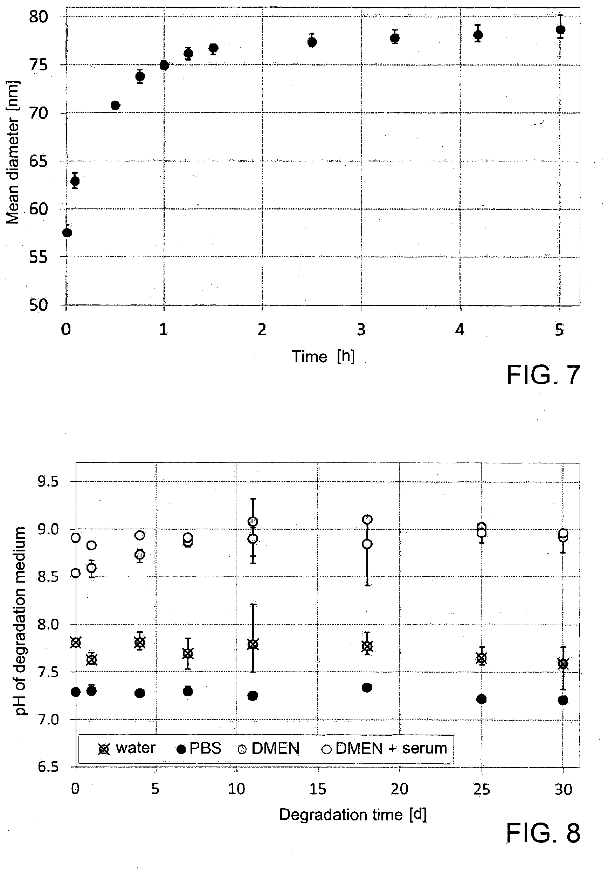

The swelling kinetics were studied from the experimental results (Figure 7) using the following power law:

$$\frac{d(t)-d(0)}{d(0)} = k_{sw} \cdot t^{n_{sw}} \tag{5}$$

in which d(t) and d(0) represent the value of the mean diameter at the generic time t and the starting value respectively, $k_{sw}$ is the swelling constant and $n_{sw}$ is the kinetic swelling order. The results obtained are $k_{sw}$ = 0.261 $\pm$ 0.020 h$^{-nsw}$ and $n_{sw}$ = 0.32 $\pm$ 0.07; the correlation between the experimental data and the theoretical model was 0.954.

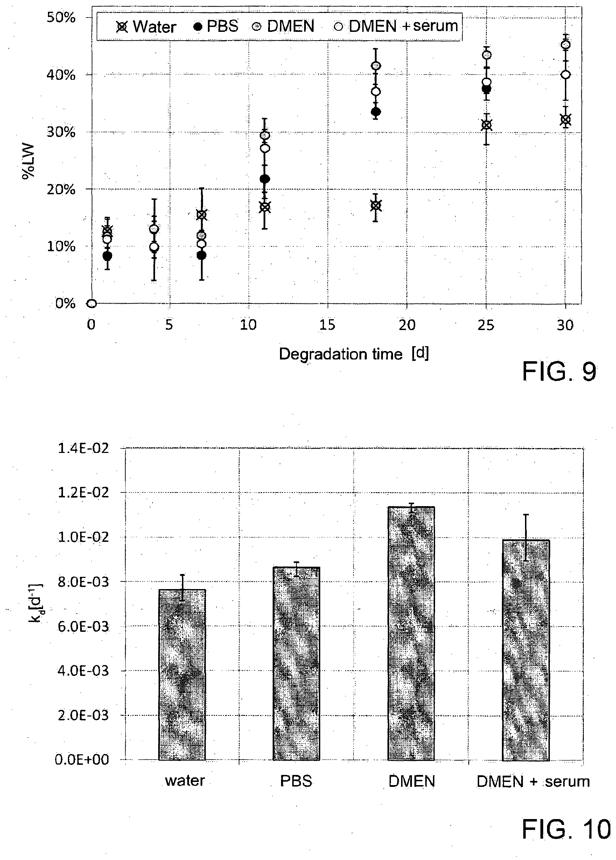

Figure 9 shows the percentage weight loss (%LW, see equation 6) observed during the degradation tests on mPEG-b-(PCL-r-PVL) nanoparticles.

$$\%LW = \frac{M(0)-M(t)}{M(0)} \cdot 100 \tag{6}$$

A mean ponderal molecular weight loss of 34.2% $\pm$ 2.15% was recorded after a month in water while the degradation was more rapid in all the other media. The polydispersion indexes (PDI), evaluated as Mw/Mn, did not significantly vary in any of the cases (data not reported).

The degradation constant $k_d$ was evaluated with the equation below:

$$M_w(t) = M_w(0) \cdot exp[-k_d \cdot t] \tag{7}$$

where $M_w(t)$ and $M_w(0)$ represent the mean ponderal molecular weight at the generic time t and the initial value respectively. Figure 10 shows the degradation constants obtained from the experimental data. The correlations between the data and the theoretical models are as follows: 0.918 (water), 0.981 (PBS), 0.954 (DMEM) and 0.947 (DMEM with serum).

Evaluation of the pH variation of the degradation medium is reported in Figure 8. During the degradation tests, the degradation products do not alter the pH of the medium.

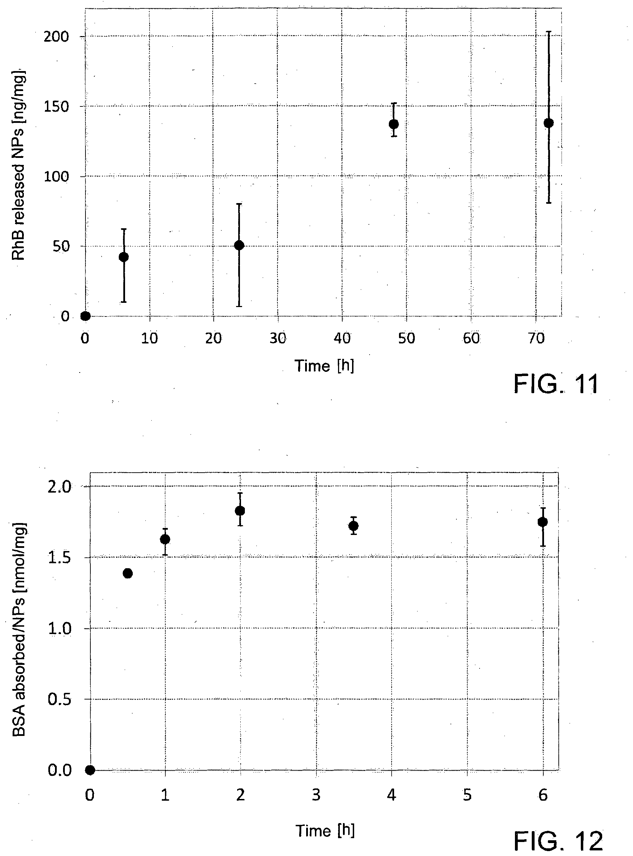

The results obtained from the RhB in vitro release test are reported in Figure 11. The release profile shows a sigmoidal trend which is typical of biodegradable release systems.

The quantity released at the end of the test is 138.0 $\pm$ 65.6 ng/mg of polymeric nanoparticles, corresponding to 24.2% of the rhodamine encapsulated.

The results obtained with the protein adsorption tests are reported in Figure 12. The adsorption kinetics were rapid in the first 2h of the experiment; after this period the phenomenon reaches a plateau value.

The quantity of protein adsorbed after 6 hours was 1.75 $\pm$ 1.73$\cdot$10$^{-1}$ nmol/mg of polymeric nanoparticles, corresponding to 23.1% of the total BSA introduced into the adsorption medium administered to the particles. The instantaneous adsorption rate of the BSA can be described by the following law:

$$q_{ads,BSA} = k_{ads} \cdot t^{n_{ads}} \tag{8}$$

where $q_{ads,BSA}$ is the quantity of BSA adsorbed (nmol) per mg of copolymer at time t, $k_{ads}$ (nmol/mg$\cdot$h$^{nads}$) and $n_{ads}$ are the adsorption constant and the kinetic order of adsorption respectively. The values of the kinetic parameters for $k_{ads}$ and $n_{ads}$ are 0.367 nmol/mg$\cdot$h$^{nads}$ and -1.60 respectively.

Example 8

Biological characterisation

All cell culture media, fetal bovine serum (FBS) and other supplements were purchased from Life Technologies (Carlsbad, CA, USA). The WST-8 cell counting kit, the dimethyl sulfoxide (DMSO), the Phalloidin-Atto633, the Hoechst colouring solution, the Triton X-100 and the paraformaldehyde were purchased from Sigma-Aldrich (St. Louis, MO, USA). The T75 flasks and the 96-well microplates were purchased from Becton Dickinson (Franklin Lakes, NJ, USA).

Immortalized endothelial cells of mice, bEND.3 (American Type Culture Collection, Manassas, VA, USA) were cultivated in a DMEM medium with high levels of glucose in the presence of 10% of FBS, L-glutamine 2 mM, 1000 U/ml penicillin and 1 mg/ml of streptomycin. The cells split from 2 to 20 times were kept in a humidified incubator for cell cultures at 37°C and 5% $CO_2$. This cell line was chosen because it is a suitable model for providing an endothelial barrier.

## Cytotoxicity assay on WST-8

The metabolic activity of bEND.3 was determined after 24h following the administration of mPEG-b-(PCL-r-PVL) nanoparticles in doses from 0.1 to 100 µg/ml using a WST-8 standard assay (Sigma) on a 96-well microplate. The cells were sown with a density of 5000 cells/well and cultivated for 24h in a humidified atmosphere at 37°C and 5% $CO_2$. 5% DMSO was used as the positive control and the culture medium was used as the negative control.

Portions of 10 µl of WST-8 reagent were added to each well for the cell count. After 3 h of incubation, the orange Formazan WST-8 was measured using a Synergy-HT (BioTEK) microplate reader at a wavelength of 460 nm. To express the cytotoxicity, the mean absorbance of the wells containing the culture medium without cells was subtracted from the mean absorbance of the control solvent, 5% DMSO or cells treated with the nanoparticles. The cell vitality percentage was calculated using the following equation:

$$\frac{Assorbanza_{trattato} - Assorbanza_{mezzo\ di\ coltura}}{Assorbanza_{controllo} - Assorbanza_{mezzo\ di\ coltura}} \times 100 \qquad (2)$$

The data were expressed as mean $\pm$ SE (standard error) of twelve replications. The statistical significance with respect to the control was calculated using one way variance analysis with the post-hoc Dunnett test (value $p < 0.05$).

## LDH release assay

The cell membrane damage was determined at 6 and 24 h after exposure of the cells to increasing concentrations of polymeric nanoparticles (doses in the range from 0.1 to 100 µg/ml administered), using the CitoTox-ONE assay (Promega, Madison, WI, USA). The assays were conducted on 96-well microplates. The cells were sown at a density of 10000 cells/well when treated with the polymeric nanoparticles (0.1 and 100 µg/ml) at a final volume of 100 µl. After 6 and 24h in humidified atmosphere at 37°C and 5% $CO_2$, the microplates were kept at controlled room temperature for half an hour, to balance at 22°C. Then, 100 µl of CitoTox-ONE reagent were added to each well. After 10 min 50 µl of Stop solution were added to each well, and the fluorescence was recorded (EX 560 nm, EM 590 nm) using a Synergy-HT (BioTEK) plate reader.

The toxicity percentage was calculated using the following equation:

$$\frac{Fluorescenza_{punto\ sperimentale} - Fluorescenza_{mezzo\ di\ coltura}}{Fluorescenza_{rilascio\ massimo\ LDH} - Fluorescenza_{mezzo\ di\ coltura}} \times 100 \qquad (3)$$

The data were expressed as mean $\pm$ SE of six replications. The statistical significance with respect to the control was calculated using one way variance analysis with the post-hoc Dunnett test (value $p < 0.05$).

## Confocal microscopy

The absorption of mPEG-b-(PCL-r-PVL) was traced with an Olympus FV100 confocal microscope. To record the cell morphology, bEND.3 cells were grown on Will-Co plates (WillCo Wlls B.V., Amsterdam, Holland) and incubated with 10 µg/ml of different formulations of mPEG-b-(PCL-r-PVL). The lysosomes were coloured with Lysotracker-red-D99 or Lysotracker-green-D99 50 nM (Life Technologies), 6, 24, 48, or 72 hours after exposure to mPEG-b-(PCL-r-PVL). The free rhodamine (113 ng/ml, the same quantity of rhodamine administered with the nanoparticles), was used as negative control for the absorption test for nanoparticles loaded with rhodamine B.

## Results

Tissue penetration of all the systemic release drugs requires interaction with the endothelial cells.

The toxicity of the polymeric nanoparticles with bEND.3 cells was then analysed to evaluate the possible damage caused by the same.

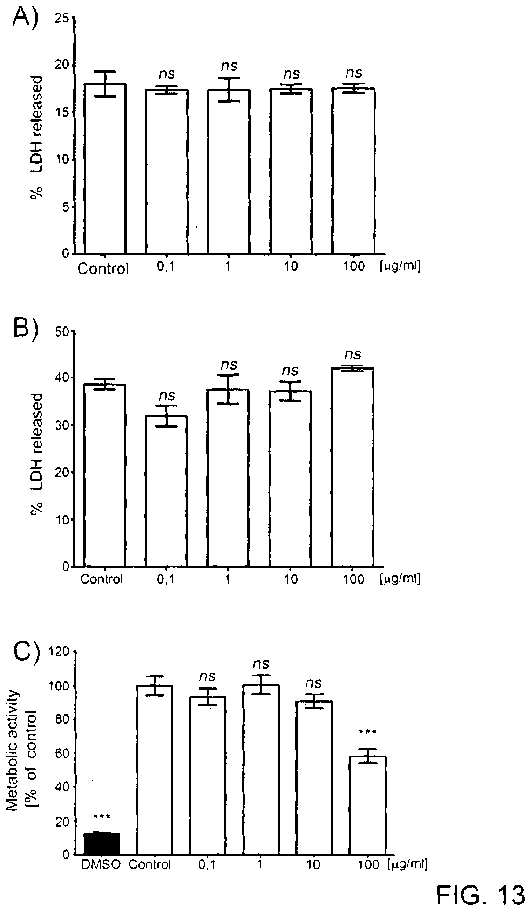

The graph of Figure 13a shows the release of lactate dehydrogenase (LDH) from the bEND.3 cells after treatment of 6h with the polymeric particles.

No damage to the membrane was found at any concentration tested. No statistically significant release of LDH was observed even after 24 h of exposure to the particles (Figure 13b). Measurement of the cell metabolism with WST-8 confirmed the previous data which show a 40% reduction only for the higher concentration of nanoparticles after 24 h (Figure 13c).

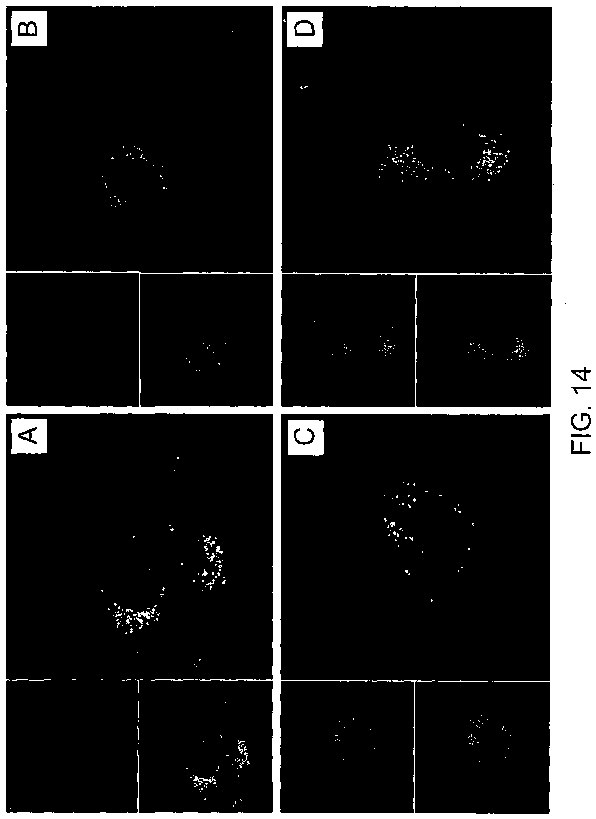

To verify the internalization of the nanoparticles by the bEND.3 cells, the mPEG-b-(PCL-r-PVL)-CF nanoparticles were incubated for 6h (Figure 14a) in a cell culture medium (10 μg/ml), then washed and analysed with confocal microscopy.

Lysotracker-red-D99 50 nM was added to mark the lysosomes and verify the intracellular localization of the nanoparticles.

The green fluorescence emitted by the nanoparticles was not detectable during this phase. However, an increase in the fluorescence of the nanoparticles and a colocalization with the lysosomes after 24, 48 and 72 h were observed (Figures 14b, 14c and 14d).

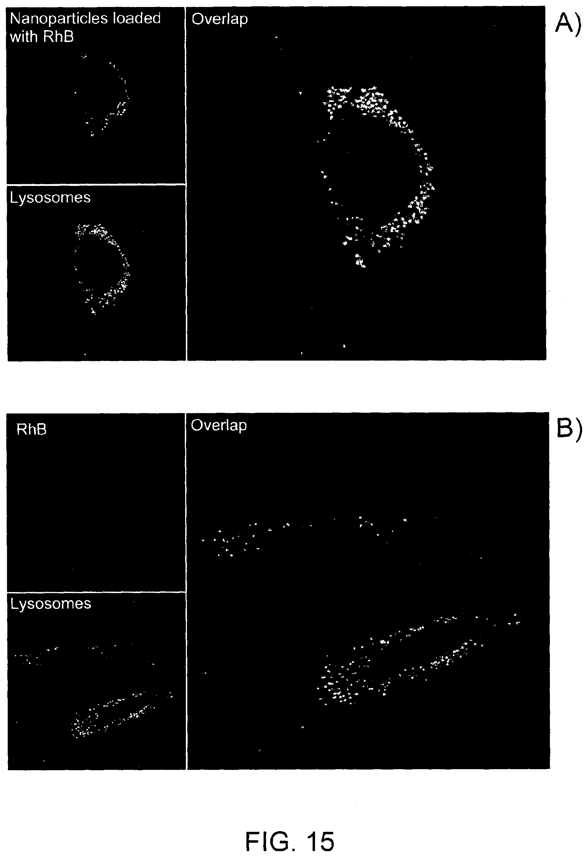

To understand the release potential by the nanoparticles, the internalization of nanoparticles loaded with RhB by bEND.3 cells was compared with the detection of fluorescent RhB not loaded on nanoparticles (Figures 15a and 15b respectively). Free RhB was not observed in the bEND.3 cells, but the nanoparticles showed the ability to effectively carry the load inside the cells.

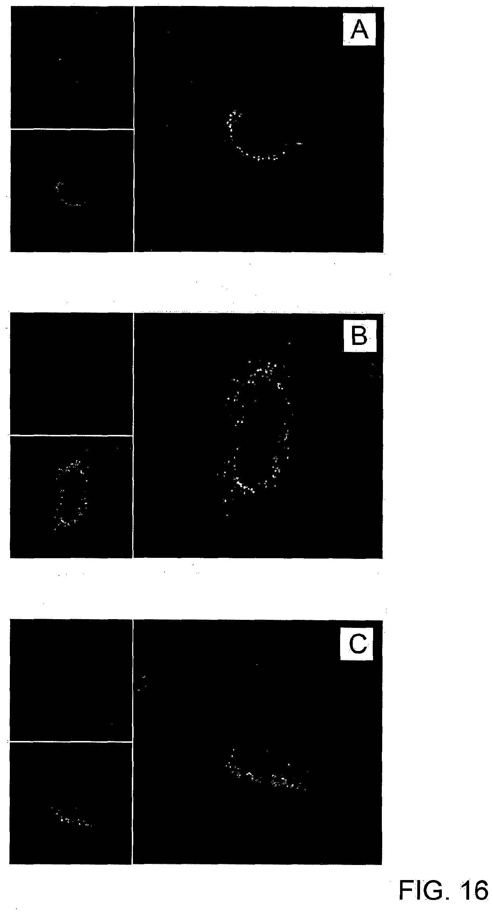

As reported in Figure 16, the RhB fluorescence signal diminishes over time suggesting that RhB has been successfully transported by the nanoparticles.

Although the RhB is colocalized with the lysosomes, its fluorescence was not detected in the cytosol.

Discussion

The copolymerization of γ-valerolactone has not been widely reported in the literature because for a long time it was considered non-polymerizable for thermodynamic reasons[27]. Zhang et Al.[29] reported the synthesis of ε-caprolactone and γ-valerolactone catalysed by boron trifluoride etherate, a dangerous catalyst with poor biocompatibility. Lin[30] conducted the same copolymerisation in the absence of catalyst, with high temperatures (200°C) and equally high reaction times (5 days). The use of a catalyst already used for the synthesis of numerous biomaterials and approved by the FDA, such as tin(II)-2-ethylhexanoate, represents an important improvement in the biocompatibility of the material with respect to the material obtained with a different catalyst and allowed the copolymer to be obtained in shorter times (4 h) and in bland conditions (120°C) compared to the synthesis without catalyst. The synthesis of selected monomers via ring opening polymerisation (ROP) using tin(II)-2-ethylhexanoate was satisfactory considering that the characterisations conducted with FTIR and NMR have shown that the copolymer obtained has the desired molar composition and molecular weight.

The molar composition of the random polyester block evaluated by means of $^1$H-NMR is 80.7/19.3, i.e. very close to the desired theoretical composition (80/20). This parameter, together with the value of the molecular weight, is correlated with the conversion into the final monomer which can be considered almost complete for both the monomers. The possibility of effectively controlling the molecular composition of the polymer is important during development of the biomaterial used for preparation of the drug transport system because it allows accurate modulation of some chemical-physical properties that characterise the end product.

A further advantage of the copolymer of the invention with the ester block composed of a chain containing -$CH_3$ groups as lateral substituents is correlated in particular with the microstructure of the end material which influences the thermal properties of the end product.

It is fundamental to study the thermal properties of the polymeric drug release systems because their degradation rate and the swelling properties, two characteristics that determine the release kinetics, depend closely on the $T_g$ and $\Delta H_m$ of the polymeric matrix.

With these characteristics, the copolymer obtained is less crystalline than the homopolymer PCL which shows a higher melting point (56.8°C) and a higher melting enthalpy (85.5 J/g)[30]. More rapid degradation kinetics are therefore obtained than those obtained with the PCL alone. It is reported that the PCL cannot be degraded by the human body due to the lack of appropriate enzymes[31]; this means that the degradation of the PCL is only a slow hydrolytic process which can be prolonged for a few years according to the molecular starting weight of the material[32,33]. For this reason, the synthesis of a less crystalline copolymer results in quicker hydrolytic degradation. The results for the degradation kinetics have shown that the degradation kinetics obtained are quicker than those of the mPEG-b-PCL copolymers (37% of molecular weight loss after 40 days[34] reported in literature).

As regards the $T_g$ values, the data measured were higher than those reported in literature for copolymers with two blocks of mPEG and PCL[35,36,37]. This can be attributed to the presence of small -$CH_3$ substituents of the valerolactone which are found near the molecule.

However, the $T_g$ recorded is lower than the incubation temperature of the degradation tests. Generally, in semicrystalline polymers, the degradation begins from the amorphous fraction of the matrix but a $T_g$ lower than the incubation temperature ensures good mobility of the chain, favouring swelling and consequently degradation. In these cases a process of annealing can occur with a reduction in the hydrolysis rate. An increase in the $T_g$ with respect to the homopolymer PCL can be unfavourable to the annealing. From the experimental tests, more rapid degradation kinetics in PBS and in a cell culture medium were observed compared to the tests conducted in water, as previously described in literature[38,39]. From the data on the pH measurements, it can be concluded that the degradation products did not alter the surrounding environment. Combining these data with the GPC analyses and in particular with the trend of the PDI values recorded during the degradation tests, it was possible to conclude that a uniform degradation occurs, which causes the loss of small oligomers from the surface of the nanoparticles but without the formation of acid degradation products which remain trapped inside the structure of the nanoparticle.

Numerous biodegradable materials such as PLGA show this behaviour which constitutes a serious limit if the drug transported is sensitive to pH variation or is even dangerous if the molecule is damaged by the acid environment.

The molecular weight of the copolymer was controlled using the mPEG as an initiator of the reaction, with the additional advantage of obtaining a PEGylated copolymer, more hydrophilic than the homopolymer poly (caprolactone) . From analysis of the static contact angle no significant differences were found between the PEGylated polymer of the invention and the homopolymer PCL. This may be correlated with a greater affinity of the PEG chains for the glass support. On the other hand, the method used for preparation of the nanoparticles may favour the formation of nanostructures with hydrophilic surface. It is known that amphiphilic block copolymers can be used for the preparation of nucleus/shell particles. In this case, the polyester block which is insoluble in EtOH forms the nucleus of the nanoparticles; the short mPEG chain is soluble in EtOH with a good affinity for this solvent and therefore forms the shell of the nanoparticles.

The technique developed for formation of the nanoparticles is based on the use of volatile solvents without the use of surfactants. The absence of surfactants, like sodium dodecyl sulphate, polysorbate 20 and Triton X-100, ensures integrity of the cell membrane and promotes the cytocompatibility of the nanoparticles.

The hydrophilicity of the surface of the nanoparticles can justify the rapid increase in the mean diameter of the nanoparticles ($\Delta$d >20% after 30 min) recorded with the DLS analyses, which represents an interesting characteristic of the system because it ensures that the drug can be released from the carrier after a short time even after degradation has taken place.

Furthermore, the increase in diameter of the nanoparticles over time in an aqueous environment can be closely correlated with the PEGylation: the hydrophilic chains linked to the surface of the nanoparticles cause an increase in absorption of the water, with a possible reduction in the quantity of blood proteins adsorbed and nanoparticle aggregation. In fact, the increase in diameter causes an increase in the concentration of the copolymer at the interface with the external environment, leading to an increase in the flow of water towards the inside followed by a repulsion of other polymeric particles and protein molecules[40]. This hypothesis was confirmed by the protein adsorption tests which showed a low tendency of the nanoparticles to bind and adsorb BSA.

Furthermore, the swelling tests have shown that the particles maintain their morphological stability after an initial phase in which an increase in diameter occurs as a consequence of absorption of the water; it has also been ascertained that the distribution of the mean diameter of the particles is unimodal and this leads to the conclusion that there is no presence of families of copolymer fragments with smaller diameters.

The morphological analysis with SEM has shown that the nanoparticles are well dispersed and have a regular spherical shape. The mean values of the diameters are in accordance with the data collected with the DLS analyses. The z potential is negative both for the native nanoparticles and the marked nanoparticles, the value recorded for the fluorescent nanoparticles is lower than the value obtained for reference nanoparticles due to the presence of the carboxyfluorescein which generated a negative potential on the surface.

Generally, negatively loaded nanoparticles do not penetrate into the cell membranes, whereas in this case the nanoparticles have been internalized due to their small diameter.

The in vitro release tests have shown that release of the RhB, a small hydrophilic molecule, follows a sigmoidal trend, with a marked increase in the release rate after the first day from the beginning of the experiment. This is a common release profile for biodegradable release systems and can be attributed to a dual drug release control: the first part of the release profile is governed by the swelling of the polymeric matrix and diffusion of the drug through the swollen copolymer; the second part of the profile is influenced by degradation of the polymeric matrix which causes release of the drug molecules.

The biological characterisation was conducted to analyse the performance of the copolymer nanoparticle and its cytocompatibility, in particular its toxicity, internalization ability and release of the drug.

The mPEG-b-(PCL-r-PVL)-CF nanoparticles do not damage the cell membranes since the release of LDH from the cells treated did not show significant differences with the non-treated control after 6 h. A slight but not significant increase in release of the LDH was recorded after 24 h of incubation in the presence of a greater quantity of nanoparticles administered (100 $\mu$g/ml). These results were confirmed by the WST-8 test which shows that there are no metabolic cell alterations for nanoparticle concentrations up to 10 $\mu$g/ml.

The results obtained show that the nanoparticles have good cytocompatibility with the bEND.3 endothelial cells in vitro. In particular, no significant toxicity induced by the nanoparticles at concentrations up to 10 $\mu$g/ml was observed. On the contrary, a marked cytotoxicity effect was observed when 100 $\mu$g/ml of a preparation of nanoparticles was administered to the cell culture. These data define a safe concentration for in vitro administration assays of mPEG-b-(PCL-r-PVL)-CF nanoparticles. The nanoparticle concentration (10 $\mu$g/ml) for performance of the cell internalization tests was chosen on the basis of these results. The high capacity of the nanocarriers to cross the cell membrane was thus demonstrated. Furthermore the complete colocalization with the lysosomes suggests a process mediated by endocytosis.

As reported, the fluorescence of the nanoparticles after 6 hours was not detected. This can be attributed not only to self-quenching of the colouring agent but also to shifting of the excitation peak of the carboxyfluorescein when conjugated with the macromolecule. It was found that the excitation peak of the marked copolymer is 468 nm instead of 492 nm of the CF colouring agent. This may limit detection of the signal during the tests. The increase in the fluorescence of the nanoparticles when the experiment time is increased can be attributed to reduction of the self-quenching of the fluorescent colouring on the surface of the nanoparticle. Probably the swelling and the surface erosion of the nanocarriers reduce the self-quenching and allow detection of the green signal for longer experimental periods. Furthermore, considering that the CF is conjugated at the tails of the macromolecules, it is reasonable to deduce that it is released following degradation, with an increase in the green fluorescence inside the lysosomes.

Example 9

Degradation behaviour

In relation to the degradation kinetics and reduction in molecular weight, the copolymer prepared as reported in example 1 has an intermediate behaviour with respect to the reference polymers and the kinetics can be modulated with the copolymeric composition. mPEG-b-(PCL-r-PVL) polymers were tested in which the CL/VL ratio was 100/0, 90/10, 80/20 and 70/30 respectively.

The tests were conducted on $2\pm0.3$ mg of polymer, placed in polypropylene vials filled with 1 ml of deionized $H_2O$ and kept in a thermostatic bath at 37°C for 33 days. At regular intervals the samples were withdrawn, the degradation medium was separated for evaluation of the pH while the degraded polymers were dried in a ventilated stove at 50°C for 3 h and then analysed by gel permeation chromatography (GPC).

For the measurement in GPC, the degraded polymers were solubilised in 250 $\mu$l of tetrahydrofuran (THF) and injected into the chromatographic apparatus, consisting of a Shimadzu chromatograph equipped with photodiode detector. Two chromatographic columns in series were used: ResiPore 3 $\mu$m 300 x 7.5 mm and PLGel 3 $\mu$m 300 x 7.5 mm (Agilent Technologies) . The mobile phase was THF (1 ml/min), the system was thermostatted at 40°C. The data obtained from the polymers of the invention were compared with those obtained for three commercial materials: poly(lactic-co-glycolic acid) (PLGA, Resomer® acid-terminated, Sigma Aldrich, LA/GA ratio: 50/50, Mw: 24-38 kDa), polycaprolactone (PCL, Sigma Aldrich, Mw: 14 kDa) and poly(3-hydroxybutyrate-co-3-hydroxyvalerate) (Sigma Aldrich, PHBHV, HB/HV ratio: 82/18, Mw: 14 kDa, calculated by GPC) .

All the tests were conducted in triplicate.

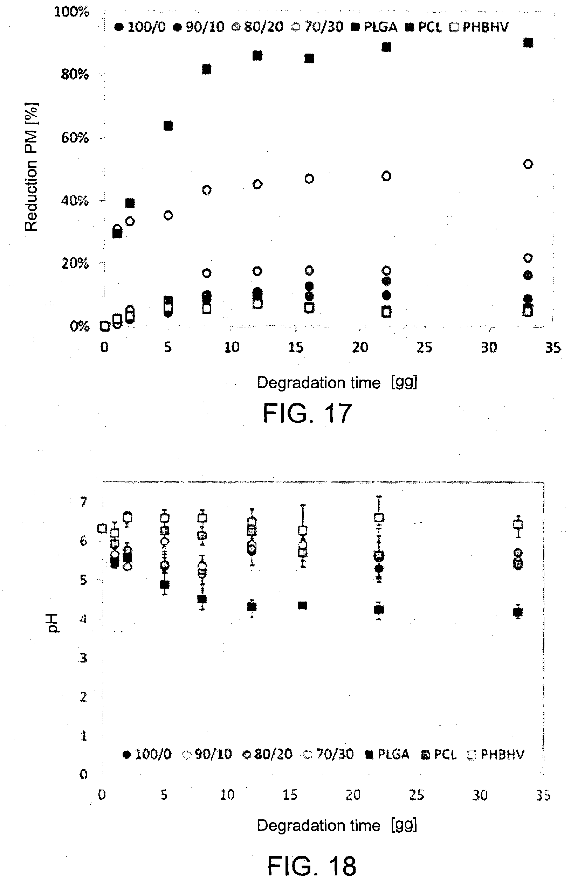

The results are illustrated in figure 17.

The results obtained, reported in figure 18, show that the copolymers containing $\gamma$-valerolactone have significantly more rapid degradation kinetics than the commercial copolymers PCL and PHBHV and also compared to the mPEG-PCL synthesis reference. Furthermore, the degradation kinetics are more rapid as the molar fraction of $\gamma$-valerolactone contained in the polymeric chain increases. The molecular weight % lowering values (Mn) obtained for the copolymer with $\varepsilon$-caprolactone/ $\gamma$-valerolactone ratio of 70/30 are comparable with those that can be obtained for the copolymer PLGA (Middleton J, Tipton A. Synthetic biodegradable polymers as orthopedic devices. Biomaterials, 2000, 21(23): 2335-2346).

Example 10

Variation in pH of the degradation medium (water) at different degradation times.

The pH measurements were conducted on the degradation means (deionized $H_2O$) after pre-set times. In particular, the degradation means were withdrawn from the vials containing the polymer, prepared as reported in example 1, during the degradation tests in the previous example and the measurement was performed using a pH-meter on the samples just taken at a temperature of 37°C.

The degradation products which form and which are released from the polymeric matrix do not cause significant reductions in the pH of the degradation means. The lower values experimentally obtained are in the range between 5.5 and 6.0 for the synthesis polymers. These values are comparable with those obtained for the commercial PCL and higher than the values obtained for the PLGA, hence a pH of approximately 4 was obtained at the end of the test. The significance of this datum for a polymer evaluated for possible use as a biomaterial is closely correlated with the biocompatibility since materials that cause significant decreases in the pH values locally can induce severe inflammatory responses in the organism. Furthermore, less acid pH values can be useful if a pH-sensitive molecule is used as the drug: acid pH values can accelerate undesired reactions which can damage the drug and its therapeutic activity.

References

1. Wang XQ, Zhang Q. (2012). pH-sensitive polymeric nanoparticles to improve oral bioavailability of peptide/protein drugs and poorly water-soluble drugs. Eur. J. Pharm. Biopharm.

2. Alexis F, Rhee JW, Richie JP, Radovic-Moreno AF, Langer R, Farokhzad OC. (2008). New frontiers in nanotechnology for cancer treatment. Urol. Oncol., 26: 74-85.

3. Torchilin V. (2009). Multifunctional and stimuli-sensitive pharmaceutical nanocarriers. Eur. J. Pharm. Biopharm., 71(3): 431-444.

4. Torchilin V. (2006). Multifunctional nanocarriers. Adv. Drug Deliv. Rev., 58(14): 1532-1555.

5. Bae Y, Jang WD, Nishiyama N, Fukushima S, Kataoka K. (2005). Multifunctional polymeric micelles with folate-mediated cancer cell targeting and pH-triggered drug releasing properties for active intracellular drug delivery. Mol. Biosyst., 1(3): 242-250.

6. Mishra B, Patel BB, Tiwari S. (2010). Colloidal nanocarriers: a review on formulation technology, types and applications toward targeted drug delivery. Nanomedicine, 6(1): 9-24.

7. Men K, Gou ML, Guo QF, Wang XH, Shi S, Kan B, Huang MJ, Luo F, Chen LJ, Zhao X, Qian ZY, Liang SF, Wei YQ. (2010) . A novel drug and gene co-delivery system based on Poly($\varepsilon$-caprolactone)-Poly(ethylene glycol)-Poly($\varepsilon$-caprolactone) grafted polyethyleneimine micelle. J. Nanosci. Nanotechnol., 10(12): 7958-7964.

8. Shi S, Zhu X, Guo Q, Wang Y, Zuo T, Luo F, Qian Z. (2012). Self-assembled mPEG-PCL-g-PEI micelles for simultaneous codelivery of chemotherapeutic drugs and DNA: synthesis and characterization in vitro. Int. J. Nanomedicine, 7: 1749-1759.

9. Shen J, Yin Q, Chen L, Zhang Z, Li Y. (2012). Co-delivery of paclitaxel and survivin shRNA by pluronic P85-PEI/TPGS complex nanoparticles to overcome drug resistance in lung cancer. Biomaterials, 33(33): 8613-8624.

10. Yadav SK, Mishra S, Mishra B. (2012). Eudragit-based nanosuspension of poorly water-soluble drug: formulation and in vitro-in vivo evaluation. AAPS PharmSciTech.

11. Feng R, Song Z, Zhai G. (2012). Preparation and in vivo pharmacokinetics of curcumin-loaded PCL-PEG-PCL triblock copolymeric nanoparticles. Int. J. Nanomedicine, 7: 4089-4098.

12. Kreuter J. (2012). Nanoparticulate systems for brain delivery of drugs. Adv. Drug. Deliv. Rev..

13. Gagliardi M, Bardi G, Bifone A. (2012). Polymeric nanocarriers for controlled and enhanced delivery of therapeutic agents to the CNS. Ther. Deliv., 3(7): 875-887.

14. Moghimi SM, Hunter AC, Murray JC. (2001). Long-circulating and target-specific nanoparticles: theory to practice. Pharmacol. Rev., (2001 Jun) 53(2):283-318.

15. Moghimi SM, Szebeni J. (2003). Stealth liposomes and long circulating nanoparticles: critical issues in pharmacokinetics, opsonization and protein-binding properties. Prog. Lipid Res., (2003 Nov) 42(6):463-78.

16. Pai SS, Tilton RD, Przybycien TM. (2009). Poly(ethylene glycol)-modified proteins: implications for poly(lactide-co-glycolide)-based microsphere delivery. AAPS J., 11(1): 88-98.

17. Avgoustakis K. (2004). Pegylated poly(lactide) and poly(lactide-co-glycolide) nanoparticles: preparation, properties and possible applications in drug delivery. Curr. Drug Deliv., 1(4): 321-333.

18. Abuchowski A, van Es T, Palczuk NC, Davis FF. (1977). Alteration of immunological properties of bovine serum albumin by covalent attachment of polyethylene glycol. J. Biol. Chem., Jun 10, 252(11):3578-81.

19. Perry JL, Reuter KG, Kai MP, Herlihy KP, Jones SW, Luft JC, Napier M, Bear JE, Desimone JM. (2012). PEGylated PRINT Nanoparticles: The Impact of PEG Density on Protein Binding, Macrophage Association, Biodistribution, and Pharmacokinetics. Nano Lett., Oct 10, 12(10):5304-10.

20. Araki T, Kono Y, Ogawara K, Watanabe T, Ono T, Kimura T, Higaki K. (2012). Formulation and evaluation of paclitaxel-loaded polymeric nanoparticles composed of polyethylene glycol and polylactic acid block copolymer. Biol. Pharm. Bull., 35(8):1306-13.

21. Brambilla D, Verpillot R, Le Droumaguet B, Nicolas J, Taverna M, Kóna J, Lettiero B, Hashemi SH, De Kimpe L, Canovi M, Gobbi M, Nicolas V, Scheper W, Moghimi SM, Tvaroška I, Couvreur P, Andrieux K. (2012). PEGylated nanoparticles bind to and alter amyloid-beta peptide conformation: toward engineering of functional nanomedicines for Alzheimer's disease. ACS Nano., Jul 24, 6(7):5897-908.

22. Szebeni J, Baranyi L, Savay S, Lutz HU, Jelezarova E, Bunger R, Alving CR. (2000). The Role of Complement Activation in Hypersensitivity to Pegylated Liposomal Doxorubicin (Doxil®). J. Liposome Res., 10(4):467-481.

23. Dutta P, Dey J, Shome A, Das PK. (2011). Nanostructure formation in aqueous solution of amphiphilic copolymers of 2-(N,N-dimethylaminoethyl)methacrylate and alkylacrylate: Characterization, antimicrobial activity, DNA binding, and cytotoxicity studies. Int. J. Pharm., 414(1-2): 298-311.

24. Barbanti SH, Santos AR Jr, Zavaglia CA, Duek EA (2004). Porous and dense poly(L-lactic acid) and poly(D,L-lactic acid-co-glycolic acid) scaffolds: in vitro degradation in culture medium and osteoblasts culture. J. Mater. Sci. Mater. Med., 15(12): 1315-1321.

25. Peng H, Ling J, Liu J, Zhu N, Ni X, Shen Z. (2010). Controlled enzymatic degradation of poly($\varepsilon$-caprolactone)-based copolymers in the presence of porcine pancreatic lipase. Polym. Deg. Stab., 95(4): 643-650.

26. Zhu Y, Gao C, Liu X, Shen J. (2002). Surface modification of polycaprolactone membrane via aminolysis and biomacromolecule immobilization for promoting cytocompatibility of human endothelial cells. Biomacromolecules, 3(6): 1312-1319.

27. You JH, Choi SW, Kim JH, Kwak YT. (2008). Synthesis and microphase separation of biodegradable poly($\varepsilon$-caprolactone)-poly(ethylene glycol)-poly(c-caprolactone) multiblock copolymer films. Macromol. Res., 16(7): 609-613.

28. Moore T, Adhikari R, Gunatillake P. (2005). Chemosynthesis of bioresorbable poly(gamma-butyrolactone) by ring-opening polymerisation: a review. Biomaterials, 26(18): 3771-3782.

29. Zhang P, Wu L, Li B. (2006). Ring-opening copolymerization of $\gamma$-valerolactone and $\varepsilon$-caprolactone catalyzed by boron trifluoride etherate. Acta Polymerica Sinica, 1(3): 510-515 (articolo in Cinese).

30. Lin WJ. (1999). Comparison of thermal characteristics and degradation properties of epsilon-caprolactone copolymers. J. Biomed. Mater. Res., 47(3): 420-423.

31. Vert M. (2009). Degradable and bioresorbable polymers in surgery and in pharmacology: beliefs and facts. J. Mater. Sci. Mater. Med., 20: 437-446.

32. Gunatillake PA, Adhikari R. (2003). Biodegradable synthetic polymers for tissue engineering. European Cells and Materials, 5: 1-16.

33. Middleton JC, Tipton AJ. (2000). Synthetic biodegradable polymers as orthopedic devices. Biomaterials, 21: 2335-2346.

34. Jiang Z, Zhu Z, Liu C, Hu Y, Wu W, Jiang X. (2008) . Non-enzymatic and enzymatic degradation of poly(ethylene glycol)-b-poly($\varepsilon$-caprolactone) diblock copolymer micelles in aqueous solution. Polymer, 49(25): 5513-5519.

35. Kang YM, Lee SH, Lee JY, Son JS, Kim BS, Lee B, Chun HJ, Min BH, Kim JH, Kim MS (2010). A biodegradable, injectable, gel system based on MPEG-b-(PCL-ran-PLLA) diblock copolymers with an adjustable therapeutic window. Biomaterials, 31(9): 2453-2460.

36. Jones DS, Djokic J, McCoy CP, Gorman SP (2002). Poly($\varepsilon$-caprolactone) and poly($\varepsilon$-caprolactone)-polyvinylpyr-rolidone-iodine blends as ureteral biomaterials: characterisation of mechanical and surface properties, degradation and resistance to encrustation in vitro. Biomaterials, 23(23): 4449-4458.

37. Endres TK, Beck-Broichsitter M, Samsonova O, Renette T, Kissel TH (2011). Self-assembled biodegradable amphiphilic PEG-PCL-lPEI triblock copolymers at the borderline between micelles and nanoparticles designed for drug and gene delivery. Biomaterials, 32(30): 7721-7731.

38. Peña J, Corrales T, Izquierdo-Barba I, Doadrio LA, Vallet-Regí M. (2006). Long term degradation of poly(3-caprolactone) films in biologically related fluids. Polymer Degradation and Stability, 91(7): 1424-1432.

39. Peña J, Corrales T, Izquierdo-Barba I, Serrano MC, Portolés MT, Pagani R, Vallet-Regí M. (2006). Alkaline-treated poly(epsilon-caprolactone) films: degradation in the presence or absence of fibroblasts. J. Biomed. Mater. Res. A., 76(4):788-97.

40. de Gennes PG. (1987). Polymers at an interface; a simplified view. Adv. Colloid Interface Sci., 27(3-4): 189-209.

41. Shin G et al. (1998). Methoxy poly(ethylene glycol)/$\varepsilon$-caprolactone amphiphilic block copolymeric micelle containing indomethacin. I. preparation and characterization. J. Contr. Rel. 51: 1-11.

42. Baimark Y (2008). Preparation of surfactant-free and core-shell type nanoparticles of Methoxy Poly(ethylene glycol)-b-Poly($\varepsilon$-caprolactone-co-d,1-lactide) diblock copolymers. As. J. Appl. Sci. 1(3): 237-245.

**Claims**

1. A block copolymer consisting of two blocks S1 and S2, wherein S1 consists of m(PEG)n, where n is an integer in the range between 4 and 50 and S2 is a random copolymer consisting of the monomer units R1 and R2, where R1 is the monomer unit corresponding to the monomer $\gamma$-valerolactone and R2 is the monomer unit corresponding to the monomer selected from the group consisting of $\varepsilon$-caprolactone, $\delta$-valerolactone, $\beta$-butyrolactone, $\varepsilon$-caprolactam, $\delta$-valerolactam.

2. A block copolymer according to claim 1, **characterized in that** R2 is $\varepsilon$-caprolactone.

3. A block copolymer according to any one of the preceding claims, **characterized in that** n is in the range between 12 and 45.

4. A block copolymer according to claim 1, **characterized in that** the molar ratio between R1 and R2 is in the range between 5/95 and 30/70.

5. A block copolymer according to any one of the preceding claims, **characterized in that** the molar ratio between S1 and S2 is in the range between 0.005 and 0.25.

6. A block copolymer according to any one of claims 1 to 5 for use in therapy or prophylaxis.

7. A method for preparing the block copolymer according to any one of the preceding claims, **characterized in that** it comprises the step of reacting R1 and R2 in the presence of S1 and of a catalyst selected from the list consisting of tin(II)-2-ethylhexanoate ($Sn(Oct)_2$), Al isopropoxide, Zn(II) lactate.

8. A method according to claim 7, **characterized in that** said catalyst is $Sn(Oct)_2$.

9. Nanoparticles comprising the block copolymer according to any one of claims 1 to 5.

10. Nanoparticles according to claim 9, **characterized in that** they have dimensions in the range between 50 nm and 320 nm.

11. Nanoparticles according to claim 9, further comprising a drug.

12. Nanoparticles according to claim 11, **characterized in that** said drug is selected from the group consisting of taxol, tacrolimus, everolimus, rapamycin, methotrexate and anthracyclines.

13. Nanoparticles according to any one of claims 11 or 12, **characterized in that** said drug is provided in amounts up to 30% by weight with respect to the copolymer.

14. A pharmaceutical composition comprising the nanoparticles according to any one of claims 11 to 13.

**Patentansprüche**

1. Blockcopolymer, das aus zwei Blöcken S1 und S2 besteht, wobei S1 aus m(PEG)n besteht, wobei n eine ganze Zahl im Bereich zwischen 4 und 50 ist, und S2 ein statistisches Copolymer ist, das aus den Monomereinheiten R1 und R2 besteht, wobei R1 die Monomereinheit ist, die dem Monomer $\gamma$-Valerolacton entspricht, und R2 die Monomereinheit ist, die dem Monomer entspricht, das ausgewählt ist aus der Gruppe bestehend aus $\epsilon$-Caprolacton, $\delta$-Valerolacton, $\beta$-Butyrolacton, $\epsilon$-Caprolactam, $\delta$-Valerolactam.

2. Blockcopolymer nach Anspruch 1, **dadurch gekennzeichnet, dass** es sich bei R2 um $\epsilon$-Caprolacton handelt.

3. Blockcopolymer nach einem der vorhergehenden Ansprüche, **dadurch gekennzeichnet, dass** n im Bereich zwischen 12 und 45 liegt.

4. Blockcopolymer nach Anspruch 1, **dadurch gekennzeichnet, dass** das Molverhältnis zwischen R1 und R2 im Bereich zwischen 5/95 und 30/70 liegt.

5. Blockcopolymer nach einem der vorhergehenden Ansprüche, **dadurch gekennzeichnet, dass** das Molverhältnis von S1 zu S2 im Bereich zwischen 0,005 und 0,25 liegt.

6. Blockcopolymer nach einem der Ansprüche 1 bis 5 zur Verwendung bei der Therapie oder Prophylaxe.

7. Verfahren zum Herstellen des Blockcopolymers nach einem der vorhergehenden Ansprüche, **dadurch gekennzeichnet, dass** es den Schritt des Umsetzens von R1 und R2 in Gegenwart von S1 und eines Katalysators, ausgewählt aus der Liste bestehend aus Zinn(II)-2-ethylhexanoat ($Sn(Oct)_2$), Aluminiumisopropoxid, Zn(II)-lactat, umfasst.

8. Verfahren nach Anspruch 7, **dadurch gekennzeichnet, dass** es sich bei dem Katalysator um $Sn(Oct)_2$ handelt.

**9.** Nanoteilchen, die das Blockcopolymer nach einem der Ansprüche 1 bis 5 umfassen.

**10.** Nanoteilchen nach Anspruch 9, **dadurch gekennzeichnet, dass** sie Abmessungen im Bereich zwischen 50 nm und 320 nm aufweisen.

**11.** Nanoteilchen nach Anspruch 9, die ferner ein Arzneimittel umfassen.

**12.** Nanoteilchen nach Anspruch 11, **dadurch gekennzeichnet, dass** das Arzneimittel ausgewählt ist aus der Gruppe bestehend aus Taxol, Tacrolimus, Everolimus, Rapamycin, Methotrexat und Anthracyclinen.

**13.** Nanoteilchen nach einem der Ansprüche 11 oder 12, **dadurch gekennzeichnet, dass** das Arzneimittel in Mengen von bis zu 30 Gew.-% bezogen auf das Gewicht des Copolymers bereitgestellt wird.

**14.** Pharmazeutische Zusammensetzung, die die Nanoteilchen nach einem der Ansprüche 11 bis 13 umfasst.


**Revendications**

**1.** Copolymère à blocs composé de deux blocs S1 et S2, dans lequel S1 est composé de m(PEG)n, où n est un entier dans la plage entre 4 et 50 et S2 est un copolymère aléatoire composé des motifs monomères R1 et R2, où R1 est le motif monomère correspondant au monomère γ-valérolactone et R2 est le motif monomère correspondant au monomère sélectionné dans le groupe composé de la ε-caprolactone, de la δ-valérolactone, de la β-butyrolactone, du ε-caprolactam, du δ-valérolactam.

**2.** Copolymère à blocs selon la revendication 1, **caractérisé en ce que** R2 est la ε-caprolactone.

**3.** Copolymère à blocs selon l'une quelconque des revendications précédentes, **caractérisé en ce que** n se situe dans la plage entre 12 et 45.

**4.** Copolymère à blocs selon la revendication 1, **caractérisé en ce que** le rapport molaire entre R1 et R2 se situe dans la plage entre 5/95 et 30/70.

**5.** Copolymère à blocs selon l'une quelconque des revendications précédentes, **caractérisé en ce que** le rapport molaire entre S1 et S2 se situe dans la plage entre 0,005 et 0,25.

**6.** Copolymère à blocs selon l'une quelconque des revendications 1 à 5 pour utilisation en thérapie ou prophylaxie.

**7.** Procédé de préparation du copolymère à blocs selon l'une quelconque des revendications précédentes, **caractérisé en ce qu'**il comprend l'étape de mise en réaction de R1 et R2 en présence de S1 et d'un catalyseur choisi dans la liste composée d'étain(II)-2-éthylhexanoate (Sn(Oct)$_2$), d'Al isopropoxyde, de Zn(II) lactate.

**8.** Procédé selon la revendication 7, **caractérisé en ce que** ledit catalyseur est le Sn(Oct)$_2$.

**9.** Nanoparticules comprenant le copolymère à blocs selon l'une quelconque des revendications 1 à 5.

**10.** Nanoparticules selon la revendication 9, **caractérisées en ce qu'**elles ont des dimensions dans la plage entre 50 nm et 320 nm.

**11.** Nanoparticules selon la revendication 9, comprenant en outre un médicament.

**12.** Nanoparticules selon la revendication 11, **caractérisées en ce que** ledit médicament est choisi dans le groupe composé du taxol, du tacrolimus, de l'évérolimus, de la rapamycine, du méthotrexate et des anthracyclines.

**13.** Nanoparticules selon l'une quelconque des revendications 11 ou 12, **caractérisées en ce que** ledit médicament est fourni dans des quantités pouvant atteindre 30 % en poids par rapport au copolymère.

**14.** Composition pharmaceutique comprenant les nanoparticules selon l'une quelconque des revendications 11 à 13.

FIG. 1

Legend (Fig. 1):
- mPEG-b-P(CL-rand-VL)   a)
- mPEG-b-P(CL-rand-VL)-CF   b)

X-axis: Wave number [cm-1] — 3950 3650 3350 3050 2750 2450 2150 1850 1550 1250 950 650

Y-axis: % T

FIG. 4

Legend (Fig. 4):
- mPEG-b-P(CL-rand-VL)   a)
- mPEG-b-P(CL-rand-VL)-CF   b)

X-axis: Retention time [min] — 8.2  8.4  8.6  8.8  9.0  9.2  9.4  9.6  9.8  10.0

Y-axis: Signal RI

FIG. 2

FIG. 3

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03093344 A1

### Non-patent literature cited in the description

- **MIDDLETON J ; TIPTON A.** Synthetic biodegradable polymers as orthopedic devices. *Biomaterials,* 2000, vol. 21 (23), 2335-2346
- **WANG XQ ; ZHANG Q.** pH-sensitive polymeric nanoparticles to improve oral bioavailability of peptide/protein drugs and poorly water-soluble drugs. *Eur. J. Pharm. Biopharm.,* 2012
- **ALEXIS F ; RHEE JW ; RICHIE JP ; RADOVIC-MORENO AF ; LANGER R ; FAROKHZAD OC.** New frontiers in nanotechnology for cancer treatment. *Urol. Oncol.,* 2008, vol. 26, 74-85
- **TORCHILIN V.** Multifunctional and stimuli-sensitive pharmaceutical nanocarriers. *Eur. J. Pharm. Biopharm.,* 2009, vol. 71 (3), 431-444
- **TORCHILIN V.** Multifunctional nanocarriers. *Adv. Drug Deliv. Rev.,* 2006, vol. 58 (14), 1532-1555
- **BAE Y ; JANG WD ; NISHIYAMA N ; FUKUSHIMA S ; KATAOKA K.** Multifunctional polymeric micelles with folate-mediated cancer cell targeting and pH-triggered drug releasing properties for active intracellular drug delivery. *Mol. Biosyst.,* 2005, vol. 1 (3), 242-250
- **MISHRA B ; PATEL BB ; TIWARI S.** Colloidal nanocarriers: a review on formulation technology, types and applications toward targeted drug delivery. *Nanomedicine,* 2010, vol. 6 (1), 9-24
- **MEN K ; GOU ML ; GUO QF ; WANG XH ; SHI S ; KAN B ; HUANG MJ ; LUO F ; CHEN LJ ; ZHAO X.** A novel drug and gene co-delivery system based on Poly($\varepsilon$-caprolactone)-Poly(ethylene glycol)-Poly($\varepsilon$-caprolactone) grafted polyethyleneimine micelle. *J. Nanosci. Nanotechnol.,* 2010, vol. 10 (12), 7958-7964
- **SHI S ; ZHU X ; GUO Q ; WANG Y ; ZUO T ; LUO F ; QIAN Z.** Self-assembled mPEG-PCL-g-PEI micelles for simultaneous codelivery of chemotherapeutic drugs and DNA: synthesis and characterization in vitro. *Int. J. Nanomedicine,* 2012, vol. 7, 1749-1759
- **SHEN J ; YIN Q ; CHEN L ; ZHANG Z ; LI Y.** Co-delivery of paclitaxel and survivin shRNA by pluronic P85-PEI/TPGS complex nanoparticles to overcome drug resistance in lung cancer. *Biomaterials,* 2012, vol. 33 (33), 8613-8624
- **YADAV SK ; MISHRA S ; MISHRA B.** Eudragit-based nanosuspension of poorly water-soluble drug: formulation and in vitro-in vivo evaluation. *AAPS PharmSciTech.,* 2012
- **FENG R ; SONG Z ; ZHAI G.** Preparation and in vivo pharmacokinetics of curcumin-loaded PCL-PEG-PCL triblock copolymeric nanoparticles. *Int. J. Nanomedicine,* 2012, vol. 7, 4089-4098
- **KREUTER J.** Nanoparticulate systems for brain delivery of drugs. *Adv. Drug. Deliv. Rev.,* 2012
- **GAGLIARDI M ; BARDI G ; BIFONE A.** Polymeric nanocarriers for controlled and enhanced delivery of therapeutic agents to the CNS. *Ther. Deliv.,* 2012, vol. 3 (7), 875-887
- **MOGHIMI SM ; HUNTER AC ; MURRAY JC.** Long-circulating and target-specific nanoparticles: theory to practice. *Pharmacol. Rev.,* June 2001, vol. 53 (2), 283-318
- **MOGHIMI SM ; SZEBENI J.** Stealth liposomes and long circulating nanoparticles: critical issues in pharmacokinetics, opsonization and protein-binding properties. *Prog. Lipid Res.,* November 2003, vol. 42 (6), 463-78
- **PAI SS ; TILTON RD ; PRZYBYCIEN TM.** Poly(ethylene glycol)-modified proteins: implications for poly(lactide-co-glycolide)-based microsphere delivery. *AAPS J.,* 2009, vol. 11 (1), 88-98
- **AVGOUSTAKIS K.** Pegylated poly(lactide) and poly(lactide-co-glycolide) nanoparticles: preparation, properties and possible applications in drug delivery. *Curr. Drug Deliv.,* 2004, vol. 1 (4), 321-333
- **ABUCHOWSKI A ; VAN ES T ; PALCZUK NC ; DAVIS FF.** Alteration of immunological properties of bovine serum albumin by covalent attachment of polyethylene glycol. *J. Biol. Chem.,* 10 June 2010, vol. 252 (11), 3578-81

- **PERRY JL ; REUTER KG ; KAI MP ; HERLIHY KP ; JONES SW ; LUFT JC ; NAPIER M ; BEAR JE ; DESIMONE JM.** PEGylated PRINT Nanoparticles: The Impact of PEG Density on Protein Binding, Macrophage Association, Biodistribution, and Pharmacokinetics. *Nano Lett.,* 10 October 2012, vol. 12 (10), 5304-10
- **ARAKI T ; KONO Y ; OGAWARA K ; WATANABE T ; ONO T ; KIMURA T ; HIGAKI K.** Formulation and evaluation of paclitaxel-loaded polymeric nanoparticles composed of polyethylene glycol and polylactic acid block copolymer. *Biol. Pharm. Bull.,* 2012, vol. 35 (8), 1306-13
- **BRAMBILLA D ; VERPILLOT R ; LE DROUMAGUET B ; NICOLAS J ; TAVERNA M ; KÓŇA J ; LETTIERO B ; HASHEMI SH ; DE KIMPE L ; CANOVI M.** PEGylated nanoparticles bind to and alter amyloid-beta peptide conformation: toward engineering of functional nanomedicines for Alzheimer's disease. *ACS Nano.,* 24 June 2012, vol. 6 (7), 5897-908
- **SZEBENI J ; BARANYI L ; SAVAY S ; LUTZ HU ; JELEZAROVA E ; BUNGER R ; ALVING CR.** The Role of Complement Activation in Hypersensitivity to Pegylated Liposomal Doxorubicin (Doxil®). *J. Liposome Res.,* 2000, vol. 10 (4), 467-481
- **DUTTA P ; DEY J ; SHOME A ; DAS PK.** Nanostructure formation in aqueous solution of amphiphilic copolymers of 2-(N,N-dimethylaminoethyl)methacrylate and alkylacrylate: Characterization, antimicrobial activity, DNA binding, and cytotoxicity studies. *Int. J. Pharm.,* 2011, vol. 414 (1-2), 298-311
- **BARBANTI SH ; SANTOS AR JR ; ZAVAGLIA CA ; DUEK EA.** Porous and dense poly(L-lactic acid) and poly(D,L-lactic acid-co-glycolic acid) scaffolds: in vitro degradation in culture medium and osteoblasts culture. *J. Mater. Sci. Mater. Med.,* 2004, vol. 15 (12), 1315-1321
- **PENG H ; LING J ; LIU J ; ZHU N ; NI X ; SHEN Z.** Controlled enzymatic degradation of poly(ε-caprolactone)-based copolymers in the presence of porcine pancreatic lipase. *Polym. Deg. Stab.,* 2010, vol. 95 (4), 643-650
- **ZHU Y ; GAO C ; LIU X ; SHEN J.** Surface modification of polycaprolactone membrane via aminolysis and biomacromolecule immobilization for promoting cytocompatibility of human endothelial cells. *Biomacromolecules,* 2002, vol. 3 (6), 1312-1319
- **YOU JH ; CHOI SW ; KIM JH ; KWAK YT.** Synthesis and microphase separation of biodegradable poly(ε-caprolactone)-poly(ethylene glycol)-poly(c-caprolactone) multiblock copolymer films. *Macromol. Res.,* 2008, vol. 16 (7), 609-613
- **MOORE T ; ADHIKARI R ; GUNATILLAKE P.** Chemosynthesis of bioresorbable poly(gamma-butyrolactone) by ring-opening polymerisation: a review. *Biomaterials,* 2005, vol. 26 (18), 3771-3782
- **ZHANG P ; WU L ; LI B.** Ring-opening copolymerization of γ-valerolactone and ε-caprolactone catalyzed by boron trifluoride etherate. *Acta Polymerica Sinica,* 2006, vol. 1 (3), 510-515
- **LIN WJ.** Comparison of thermal characteristics and degradation properties of epsilon-caprolactone copolymers. *J. Biomed. Mater. Res.,* 1999, vol. 47 (3), 420-423
- **VERT M.** Degradable and bioresorbable polymers in surgery and in pharmacology: beliefs and facts. *J. Mater. Sci. Mater. Med.,* 2009, vol. 20, 437-446
- **GUNATILLAKE PA ; ADHIKARI R.** Biodegradable synthetic polymers for tissue engineering. *European Cells and Materials,* 2003, vol. 5, 1-16
- **MIDDLETON JC ; TIPTON AJ.** Synthetic biodegradable polymers as orthopedic devices. *Biomaterials,* 2000, vol. 21, 2335-2346
- **JIANG Z ; ZHU Z ; LIU C ; HU Y ; WU W ; JIANG X.** Non-enzymatic and enzymatic degradation of poly(ethylene glycol)-b-poly(ε-caprolactone) diblock copolymer micelles in aqueous solution. *Polymer,* 2008, vol. 49 (25), 5513-5519
- **KANG YM ; LEE SH ; LEE JY ; SON JS ; KIM BS ; LEE B ; CHUN HJ ; MIN BH ; KIM JH ; KIM MS.** A biodegradable, injectable, gel system based on MPEG-b-(PCL-ran-PLLA) diblock copolymers with an adjustable therapeutic window. *Biomaterials,* 2010, vol. 31 (9), 2453-2460
- **JONES DS ; DJOKIC J ; MCCOY CP ; GORMAN SP.** Poly(ε-caprolactone) and poly(ε-caprolactone)-polyvinylpyrrolidone-iodine blends as ureteral biomaterials: characterisation of mechanical and surface properties, degradation and resistance to encrustation in vitro. *Biomaterials,* 2002, vol. 23 (23), 4449-4458
- **ENDRES TK ; BECK-BROICHSITTER M ; SAMSONOVA O ; RENETTE T ; KISSEL TH.** Self-assembled biodegradable amphiphilic PEG-PCL-IPEI triblock copolymers at the borderline between micelles and nanoparticles designed for drug and gene delivery. *Biomaterials,* 2011, vol. 32 (30), 7721-7731
- **PEÑA J ; CORRALES T ; IZQUIERDO-BARBA I ; DOADRIO LA ; VALLET-REGÍ M.** Long term degradation of poly(3-caprolactone) films in biologically related fluids. *Polymer Degradation and Stability,* 2006, vol. 91 (7), 1424-1432
- **PEÑA J ; CORRALES T ; IZQUIERDO-BARBA I ; SERRANO MC ; PORTOLÉS MT ; PAGANI R ; VALLET-REGÍ M.** Alkaline-treated poly(epsilon-caprolactone) films: degradation in the presence or absence of fibroblasts. *J. Biomed. Mater. Res. A.,* 2006, vol. 76 (4), 788-97
- **DE GENNES PG.** Polymers at an interface; a simplified view. *Adv. Colloid Interface Sci.,* 1987, vol. 27 (3-4), 189-209

- **SHIN G et al.** Methoxy poly(ethylene glycol)/$\varepsilon$-caprolactone amphiphilic block copolymeric micelle containing indomethacin. I. preparation and characterization. *J. Contr. Rel.,* 1998, vol. 51, 1-11

- **BAIMARK Y.** Preparation of surfactant-free and core-shell type nanoparticles of Methoxy Poly(ethylene glycol)-b-Poly($\varepsilon$-caprolactone-co-d,1-lactide) diblock copolymers. As. *J. Appl. Sci.,* 2008, vol. 1 (3), 237-245